(19) Europäisches Patentamt
European Patent Office
Office européen des brevets

(11) EP 3 503 923 B1

(12) **EUROPEAN PATENT SPECIFICATION**

(45) Date of publication and mention of the grant of the patent:
**04.10.2023 Bulletin 2023/40**

(21) Application number: **17761701.6**

(22) Date of filing: **23.08.2017**

(51) International Patent Classification (IPC):
***A61K 45/06*** (2006.01) ***A61K 31/506*** (2006.01)
***A61K 31/445*** (2006.01) ***A61K 31/519*** (2006.01)
***A61K 31/405*** (2006.01) ***A61P 35/00*** (2006.01)

(52) Cooperative Patent Classification (CPC):
(C-Sets available)
**A61K 31/506; A61K 31/445; A61K 31/519; A61K 45/06; A61P 35/00** (Cont.)

(86) International application number:
**PCT/US2017/048183**

(87) International publication number:
**WO 2018/039324 (01.03.2018 Gazette 2018/09)**

(54) **COMBINATION THERAPIES FOR THE TREATMENT OF HEPATOCELLULAR CARCINOMA**

KOMBINATIONSTHERAPIE ZUR BEHANDLUNG VON HEPATOZELLULÄREM KARZINOM

THÉRAPIES COMBINÉES DESTINÉE AU TRAITEMENT DU CANCER HÉPATOCELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **23.08.2016 US 201662378455 P**

(43) Date of publication of application:
**03.07.2019 Bulletin 2019/27**

(73) Proprietor: **Eisai R&D Management Co., Ltd.**
**Tokyo 112-8088 (JP)**

(72) Inventors:
- **SELVARAJ, Anand**
  **Cambridge**
  **Massachusetts 02139 (US)**
- **SMITH, Peter**
  **Arlington**
  **Massachusetts 02474 (US)**

(74) Representative: **Hoffmann Eitle**
**Patent- und Rechtsanwälte PartmbB**
**Arabellastraße 30**
**81925 München (DE)**

(56) References cited:
**WO-A1-2011/071821 WO-A1-2011/130232**
**WO-A1-2013/006368 WO-A1-2015/057938**
**WO-A1-2016/025650 WO-A1-2016/168331**

- **RIVADENEIRA D B ET AL: "Proliferative Suppression by CDK4/6 Inhibition: Complex Function of the Retinoblastoma Pathway in Liver Tissue and Hepatoma Cells", GASTROENTEROLOGY, ELSEVIER, US, vol. 138, no. 5, 1 May 2010 (2010-05-01), pages 1920-1930.e2, XP027089071, ISSN: 0016-5085 [retrieved on 2010-01-25]**
- **GUAGNANO V ET AL: "Discovery of 3-(2,6-Dichloro-3,5-dimethoxy-phenyl)-1-{6 -[4-(4-ethyl-piperazin-1-yl)-phenylamino]-pyrimidin-4-yl}-1-methyl-urea (NVP-BGJ398), A Potent and Selective Inhibitor of the Fibroblast Growth Factor Receptor Family of Receptor Tyrosine Kinase", JOURNAL OF MEDICINAL CHEMISTRY, AMERICAN CHEMICAL SOCIETY, US, vol. 54, no. 20, 27 October 2011 (2011-10-27), pages 7066-7083, XP002689272, ISSN: 0022-2623, DOI: 10.1021/JM2006222 [retrieved on 2011-09-21]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 503 923 B1

- **Lucia Nogova ET AL: "Evaluation of BGJ398, a Fibroblast Growth Factor Receptor 1-3 Kinase Inhibitor, in Patients With Advanced Solid Tumors Harboring Genetic Alterations in Fibroblast Growth Factor Receptors: Results of a Global Phase I, Dose-Escalation and Dose-Expansion Study", Journal of Clinical Oncology, vol. 35, no. 2, 10 January 2017 (2017-01-10), pages 157-165, XP055606927, US ISSN: 0732-183X, DOI: 10.1200/JCO.2016.67.2048**

(52) Cooperative Patent Classification (CPC): (Cont.)

C-Sets
**A61K 31/445, A61K 2300/00;**
**A61K 31/506, A61K 2300/00;**
**A61K 31/519, A61K 2300/00**

## Description

BACKGROUND

Liver cancer is the second greatest cause of mortality from any type of cancer, and the 16th most common cause of death worldwide (Llovet JM, et al., 2015 "Advances in targeted therapies for hepatocellular carcinoma in the genomic era." Nat. Rev. Clin Oncology 12, 408-424). Hepatocellular carcinoma (HCC) accounts for up to 90% of all primary liver cancers (Llovet JM *et al.* 2015).

Various signaling pathways have been implicated in HCC, including fibroblast growth factors (FGF) (particularly FGF19/FGFR4), epidermal growth factor (EGF), vascular endothelial growth factor (VEGF), ERK/MAPK, and mechanistic target of rapamycin (mTOR), among others (Llovet JM *et al.,* 2015). FGF19 is overexpressed in about a third of all HCC, and this overexpression is hypothesized to hyperactivate FGFR4 and its downstream signaling pathway leading to enhanced tumor growth (Xie MH et al., 1999 "FGF-19, a novel fibroblast growth factor with unique specificity for FGFR4." Cytokine. 1999 Oct;11(10):729-35; Sawey, et al., 2011 "Identification of a therapeutic strategy targeting amplified FGF19 in liver cancer by Oncogenomic screening." Cancer Cell. 2011 Mar 8;19(3):347-58.). Similar to FGF19/FGFR4 pathway, the CDK4/6 pathway activation is also involved in HCC pathogenesis (Rivadeneira et al., 2010 "Proliferative Suppression by CDK4/6 Inhibition: Complex Function of the Retinoblastoma Pathway in Liver Tissue and Hepatoma Cells." Gastroenterology 138:1920-1930).

Compound 1 is a selective, orally bioavailable small molecule FGFR4 inhibitor with the structure shown in Formula I, and the chemical name *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide:

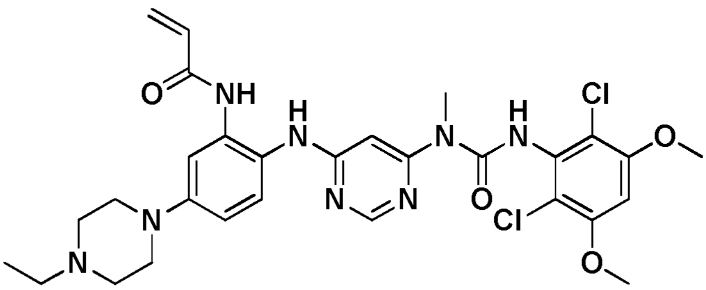

(I).

Compound 1 and its synthesis are reported in PCT International Application Publication No. WO2015/057938, published on April 23, 2015.

Palbociclib (6-acetyl-8-cyclopentyl-5-methyl-2-{[5-(piperazin-1-yl)pyridin-2-yl]amino}pyrido[2,3-*d*]pyrimidin-7(8*H*)-one) is an FDA-approved inhibitor of cyclin-dependent kinase (CDK) 4 and 6. Palbociclib has the following structure:

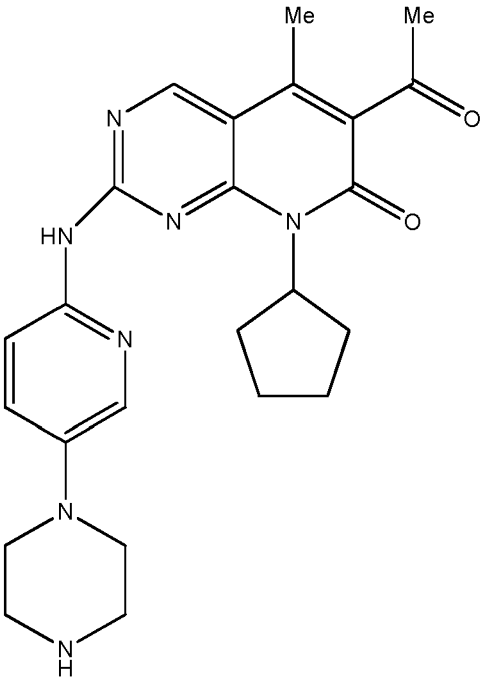

See U.S. Patent Nos. 6,936,612; 7,208,489, and 7,456,168.

Ribociclib (7-Cyclopentyl-2-(5-piperazin-1-yl-pyridin-2-ylamino)-7H-pyrrolo[2,3-d]pyrimidine-6-carboxylic acid dimethylamide) is an FDA-approved inhibitor of cyclin-dependent kinase (CDK) 4 and 6. Ribociclib has the following structure:

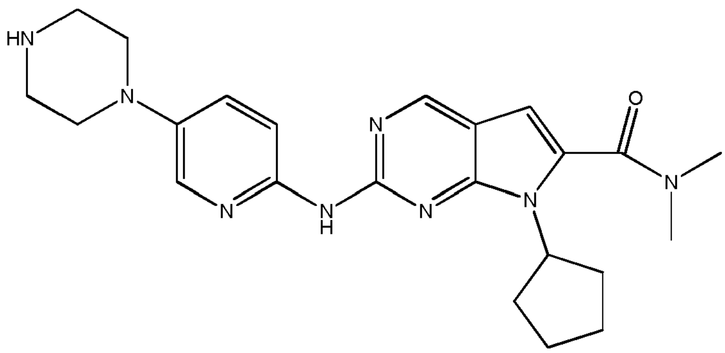

See U.S. Patent App. Pub. No. US20120115878, PCT Publication No. WO2007140222, PCT Publication No. WO2012061156; PCT Publication No. WO2011130232; PCT Publication No. WO2011101417.

Abemaciclib is an inhibitor of CDK 4/6 with the name *N*-(5-((4-ethylpiperazin-1-yl)methyl)pyridin-2-yl)-5-fluoro-4-(4-fluoro-1-isopropyl-2-methyl-*1H*-benzo[d]imidazol-6-yl)pyrimidin-2-amine. Abemaciclib has the following structure:

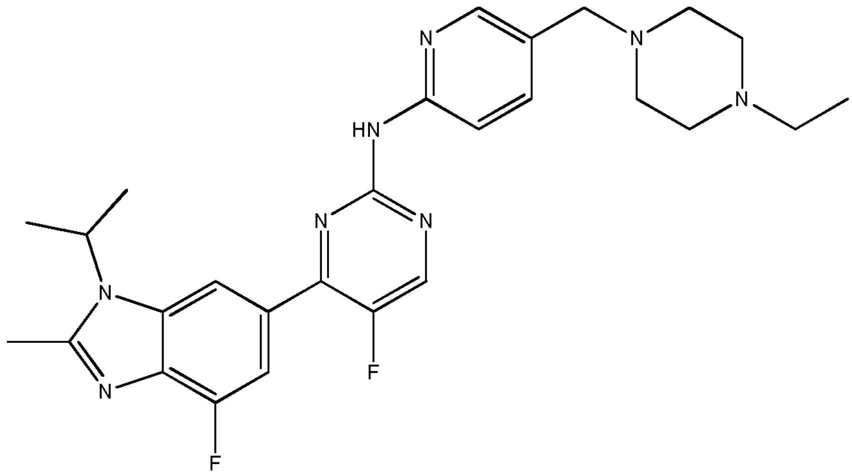

.

See O'Leary, et al., "Treating Cancer with Selective CDK 4/6 Inhibitors" Nat. Rev. (Published Online March. 31, 2016); PCT Publication No. WO2016110224, United States Patent App. Pub. No. 20100160340; and PCT Publication No. WO2016025650.

G1T-38 (also referred to as GZ-38-1 or G1T38-1) is a reported inhibitor of CDK 4/6. G1T-38, which is studied by G1 Therapeutics, Inc., of Research Triangle Park, North Carolina, is reported in Abstract #2824 of the 2016 AACR Annual Meeting, held April 16-20 in New Orleans, Louisiana, entitled "G1T38, A Novel, Oral, Potent and Selective CDK 4/6 Inhibitor for the Treatment of RB Competent Tumors," by J. Sorrentino, J. Bisi, P. Roberts, and J. Strum. G1T-38 has the chemical name 2'-((5-(4-isopropylpiperazin-1-yl)pyridin-2-yl)amino)-7', 8'dihydro-6'Hspiro[cyclohexane1,9' pyrazino[1',2':1,5] pyrrolo[2,3-d]pyrimidin]-6'-one, and the structure set forth below:

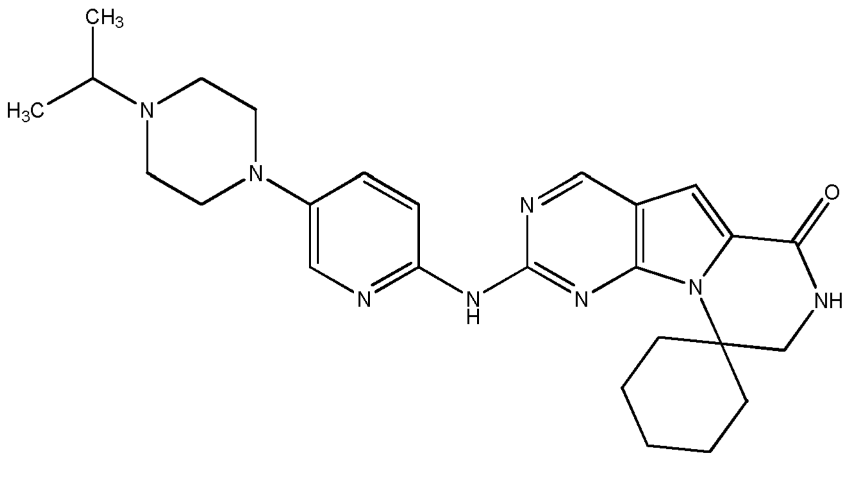

See Bisi, et al., "Preclinical development of G1T38: A novel, potent and selective inhibitor of cyclin dependent kinases 4/6 for use as an oral antineoplastic in patients with CDK4/6 sensitive tumors," Oncotarget, Advance Publications 2017 (March 15, 2017).

G1T-28 is an inhibitor of CDK 4/6 with the name 2'-((5-(4-methylpiperazin-1-yl)pyridin-2-yl)amino)-7',8'-dihydro-6'H-spiro[cyclohexane-1,9'-pyrazino[1',2':1,5]pyrrolo[2,3-d]pyrimidin]-6'-one. G1T-28 has the following structure:

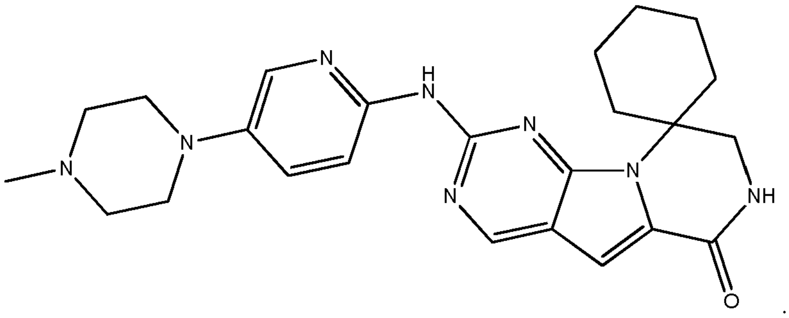

See, for example, Bisi, et al., "Preclinical Characterization of G1T28: A Novel CDK4/6 Inhibitor for Reduction of Chemotherapy-induced Myelosuppression" Mol. Cancer Ther.; 15(5) 783-93, May 2016; U.S. Patent Application Publication No. US20160220569; PCT International Patent Application Publication Nos. WO2014144326; WO2014144847; and WO2016040848.

AT-7519 is an inhibitor of CDK 4/6 with the name N-(4-piperidinyl)-4-(2,6-dichlorobenzoylamino)-1*H*-pyrazole-3 carboxamide. AT-7519 has the following structure:

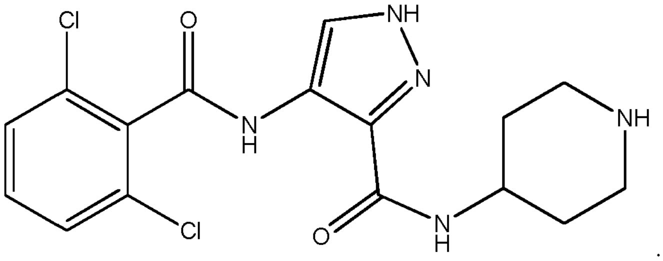

See, for example, PCT International Patent Application Publication Nos. WO 2005012256; WO 2006077424; WO 2006077426; WO 2008001101; WO 2006077425; WO 2006077428; WO 2008007113; WO 2008007122; and WO 2008009954

FLX-925 (also known as AMG-925) is an inhibitor of CDK 4/6 with the name 2-Hydroxy-1-[2-[[9-(trans-4-methylcyclohexyl)-9*H*-pyrido[4',3':4,5]pyrrolo[2,3-d]pyrimidin-2-yl]amino]-7,8-dihydro-1,6-naphthyridin-6(5*H*)-yl]ethanone FLX-925 has the following structure:

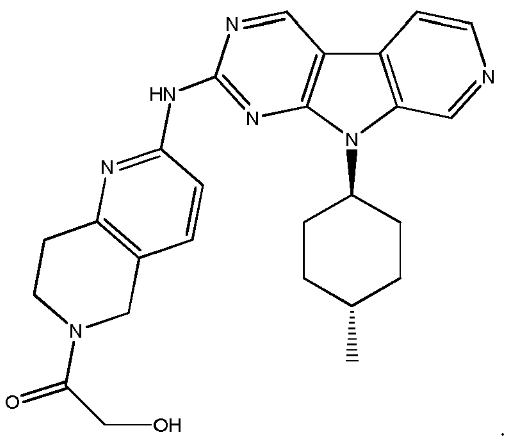

See, for example, U.S. Patent Application Pub. No. 2014163052 and PCT International Patent Application Publication No. WO 2012129344.

Alvocidib is an inhibitor of CDK 4/6 with the name 2-(2-chlorophenyl)-5,7-dihydroxy-8-((3*S*,4*R*)-3-hydroxy-1-methylpiperidin-4-yl)-4*H*-chromen-4-one. Alvocidib has the following structure:

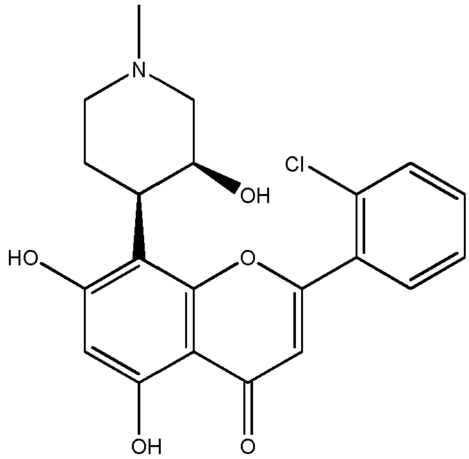
.

See, for example, U.S. Patent Application Publication No. US2011189175 and US2011189175; PCT International Patent Application Publication Nos. WO 2000044362; WO 2001041747; WO 2001053293; WO 2001053294; WO 2002022133; WO 2007010946,.

Despite advances in the treatment of HCC, there is a need to provide improved treatment for HCC. Despite advances in the treatment of IHCC, there is a need to provide improved treatment for IHCC.

SUMMARY

The present invention provides a combination therapy, comprising an effective amount of Compound 1 or a pharmaceutically acceptable salt thereof and an effective amount of a CDK4/6 inhibitor or a pharmaceutically effective amount thereof for use in threating hepatocellular carcinoma in a patient in need thereof.

The invention is set out in the appended set of claims.

BRIEF DESCRIPTION OF THE DRAWINGS

FIG. 1A through FIG. 1C show antitumor effects of Compound 1 and CDK 4/6 inhibitor ribociclib in the JHH7 xenograft model of hepatocellular carcinoma. Both Compound 1 and ribociclib, each as a free base, were given orally (PO) once daily (QD) for 8 days. Data represent the mean ±SEM for tumor volume.

FIG. 1A shows Ribociclib as single agent (****P ≤ 0.0001 compared to vehicle controls using two way ANOVA followed by Sidak post hoc test).

FIG. 1B shows two dose levels of Ribociclib in combination with 300mg/kg Compound 1 (****P ≤ 0.0001 compared to 300mg/kg Compound 1 single agent group using two way ANOVA followed by Sidak post hoc test).

FIG. 1C shows two dose levels of Ribociclib in combination with 500mg/kg Compound 1 (****P ≤ 0.0001 for 500mg/kg Compound 1 single agent group in comparison to the vehicle controls and ****P ≤ 0.0001 for combinations compared to 500mg/kg Compound 1 single agent group using two way ANOVA followed by Sidak post hoc test).

FIG. 2A through FIG. 2C show antitumor effects of Compound 1 and CDK 4/6 inhibitor palbociclib in the JHH xenograft model of hepatocellular carcinoma. Both Compound 1 and Palbociclib, each as a free base, were given orally (PO) once daily (QD) for 8 days. Data represent the mean ±SEM for Tumor Volume.

FIG. 2A shows palbociclib as single agent (*P ≤ 0.05 compared to vehicle controls using two way ANOVA followed by Sidak post hoc test).

FIG. 2B shows two dose levels of palbociclib in combination with 300mg/kg Compound 1 (**P ≤ 0.01 compared to vehicle control and ****P ≤ 0.0001 compared to 300mg/kg Compound 1 single agent group using two way ANOVA followed by Sidak post hoc test).

FIG. 2C shows two dose levels of palbociclib in combination with 500mg/kg Compound 1 (****P ≤ 0.0001 for 500mg/kg Compound 1 single agent group in comparison to the vehicle controls and ****P ≤ 0.0001 for combinations compared to 500mg/kg Compound 1 single agent group using two way ANOVA followed by Sidak post hoc test).

FIG. 3A and FIG. 3B show antitumour effects of Compound 1 and CDK 4/6 inhibitor palbociclib in the LIX066 patient derived xenograft model of hepatocellular carcinoma. Both Compound 1 and Palbociclib were given orally (PO) once daily (QD) for 8 days. Data represent the mean ±SEM for Tumor Volume.

FIG. 3A shows Compound 1 300mg/kg either as single agent or in combination with 100mg/kg palbociclib (***P ≤

0.001 for palbociclib 100mg/kg single agent compared to vehicle controls, ****P ≤ 0.0001 for 300mg/kg Compound 1 single agent group in comparison to the vehicle controls and ****P ≤ 0.0001 for combinations compared to 300mg/kg Compound 1 single agent group using two way ANOVA followed by Sidak post hoc test).

FIG. 3B shows Compound 1 500mg/kg either as single agent or in combination with 100mg/kg palbociclib (***P ≤ 0.001 for palbociclib 100mg/kg single agent compared to vehicle controls, ****P ≤ 0.0001 for 500mg/kg Compound 1 single agent group in comparison to the vehicle controls and ****P ≤ 0.0001 for combinations compared to 500mg/kg Compound 1 single agent group using two way ANOVA followed by Sidak post hoc test).

DETAILED DESCRIPTION OF EMBODIMENTS

Provided herein are combination therapies useful in treating hepatocellular carcinoma (HCC) and intrahepatic cholangiocarcinoma (IHCC). In some embodiments, the combination therapies include administration of Compound 1 in combination with a CDK 4/6 inhibitor. In certain embodiments the CDK 4/6 inhibitor is Palbociclib and pharmaceutically acceptable salts thereof. In other embodiments, the CDK 4/6 inhibitor is ribociclib. In still other embodiments the CDK 4/6 inhibitor is abemaciclib and pharmaceutically acceptable salts thereof..

Provided herein are combinations of therapeutic agents for use in treating hepatocellular carcinoma. As used herein, a "combination of therapeutic agents" and similar terms refer to a combination of two types of therapeutic agents: (1) Compound 1 and/or pharmacologically active salts thereof and (2) a CDK 4/6 inhibitor, and/or pharmacologically active salts thereof. "Combination" as used herein (including in the term "combination of therapeutic agents") refers to these types of therapeutic agents co-formulated in a single dosage form, individually formulated and co-administered, or individually formulated and sequentially administered.

Compound 1 is a selective, orally bioavailable small molecule FGFR4 inhibitor with the structure shown in Formula I:

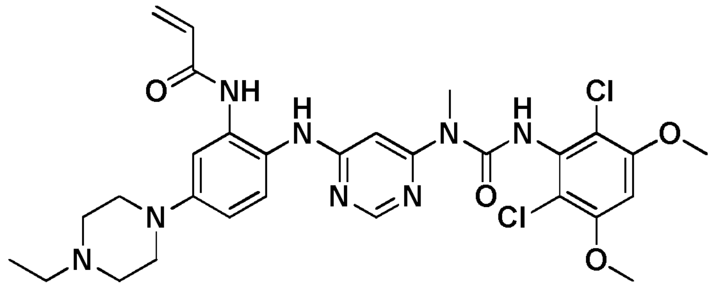

(I).

Compound 1 and its synthesis are reported in PCT International Application Publication No. WO2015/057938, published on April 23, 2015. When used in combinations as described herein, Compound 1 may be administered to patients in any of the following daily dosage amounts: 150 mg, 300 mg, 600 mg, 1000 mg, 1500 mg or 2000 mg. The daily dosage amount may be from 50 mg to 3000 mg, from 50 mg to 600 mg, or from 200 mg to 400 mg. The daily dosage may be part of a cyclic regimen lasting 14 days or 21 days. The daily dosage amount may be administered as a single dosage or as multiple dosages.

CDK 4/6 inhibitors suitable for use herein may include, for example, ribociclib, palbociclib, and abemaciclib, and their pharmaceutically acceptable salts and hydrates.

Administration of a combination of therapeutic agents comprises administration of the individual therapeutic agents in combination in a single formulation or unit dosage form, administration of the individual therapeutic agents of the combination concurrently but separately, or administration of the individual agents of the combination sequentially by any suitable route. The dosage of the individual therapeutic agents of the combination may require more frequent administration of one of the agents as compared to the other agent in the combination. Therefore, to permit appropriate dosing, packaged pharmaceutical products may contain one or more dosage forms that contain the combination of agents, and one or more dosage forms that contain one of the combinations of agents, but not the other agent(s) of the combination.

Combinations as reported herein may include embodiments wherein one or more of Compound 1 and a CDK 4/6 inhibitor are administered as a pharmaceutically acceptable salt or as a free base. There is no requirement that both compounds be administered as the same pharmaceutically acceptable salt, but they may be. In particular embodiments combinations comprise a free base form of Compound 1 and a free base form of CDK 4/6 inhibitor. In other embodiments combinations may comprise an HCl form of Compound 1 and a CDK 4/6 inhibitor. In some embodiments the CDK 4/6 inhibitor may be a free base. In some embodiments the CDK 4/6 inhibitor may be a pharmaceutically acceptable salt. In some embodiments the CDK 4/6 inhibitor may be a hydrate.

"Pharmaceutically acceptable salt" as used herein refers to acid addition salts or base addition salts of the compounds in the present disclosure. A pharmaceutically acceptable salt is any salt which retains the activity of the parent compound and does not impart any unduly deleterious or undesirable effect on a subject to whom it is administered and in the context in which it is administered. Pharmaceutically acceptable salts includemetal complexes and salts of both inorganic and carboxylic acids. Pharmaceutically acceptable salts also include metal salts such as aluminum, calcium, iron, magnesium, manganese and complex salts. In addition, pharmaceutically acceptable salts includeacid salts such as acetic, aspartic, alkylsulfonic, arylsulfonic, axetil, benzenesulfonic, benzoic, bicarbonic, bisulfuric, bitartaric, butyric, calcium edetate, camsylic, carbonic, chlorobenzoic, citric, edetic, edisylic, estolic, esyl, esylic, formic, fumaric, gluceptic, gluconic, glutamic, glycolic, glycolylarsanilic, hexamic, hexylresorcinoic, hydrabamic, hydrobromic, hydrochloric, hydroiodic, hydroxynaphthoic, isethionic, lactic, lactobionic, maleic, malic, malonic, mandelic, methanesulfonic, methylnitric, methylsulfuric, mucic, muconic, napsylic, nitric, oxalic, p-nitromethanesulfonic, pamoic, pantothenic, phosphoric, monohydrogen phosphoric, dihydrogen phosphoric, phthalic, polygalactouronic, propionic, salicylic, stearic, succinic, sulfamic, sulfanlic, sulfonic, sulfuric, tannic, tartaric, teoclic, toluenesulfonic.

Embodiments may be hydrochloride salts. Pharmaceutically acceptable salts may be derived from amino acids includingcysteine. Methods for producing compounds as salts are known to those of skill in the art (*see, e.g.,* Stahl et al., Handbook of Pharmaceutical Salts: Properties, Selection, and Use, Wiley-VCH; Verlag Helvetica Chimica Acta, Zurich, 2002; Berge et al., J. Pharm. Sci. 66: 1, 1977).

An "effective amount" of a combination of therapeutic agents (e.g., Compound 1 and a CDK 4/6 inhibitor) is an amount sufficient to provide an observable therapeutic benefit compared to HCC or IHCC left untreated in a subject or patient.

Active agents as reported herein can be combined with a pharmaceutically acceptable carrier to provide pharmaceutical formulations thereof. The particular choice of carrier and formulation will depend upon the particular route of administration for which the composition is intended.

"Pharmaceutically acceptable carrier" as used herein refers to a nontoxic carrier, adjuvant, or vehicle that does not destroy the pharmacological activity of the compound with which it is formulated. Pharmaceutically acceptable carriers, adjuvants or vehicles that may be used in the compositions of this invention includesorbic acid, potassium sorbate, partial glyceride mixtures of saturated vegetable fatty acids, water, salts or electrolytes, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene glycol and wool fat.

The compositions of the present invention may be suitable for parenteral, oral, inhalation spray, topical, rectal, nasal, buccal, vaginal or implanted reservoir administration, etc. In some embodiments, the formulation comprises ingredients that are from natural or non-natural sources. In some embodiments, the formulation or carrier may be provided in a sterile form. Examples of a sterile carrier include endotoxin-free water or pyrogen-free water.

The term "parenteral" as used herein includes subcutaneous, intravenous, intramuscular, intra-articular, intra-synovial, intrasternal, intrathecal, intrahepatic, intralesional and intracranial injection or infusion techniques. In particular embodiments, the compounds are administered intravenously, orally, subcutaneously, or via intramuscular administration. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a nontoxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids and their glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents that are commonly used in the manufacture of pharmaceutically acceptable solid, liquid, or other dosage forms may also be used for the purposes of formulation.

For oral administration, a compound or salt may be provided in an acceptable oral dosage form, including, capsules, tablets, aqueous suspensions or solutions. In the case of tablets for oral use, carriers commonly used include lactose and corn starch. Lubricating agents, such as magnesium stearate, may also be added. For oral administration in a capsule form, useful diluents include lactose and dried cornstarch. When aqueous suspensions are required for oral use, the active ingredient may be combined with emulsifying and suspending agents. If desired, certain sweetening, flavoring or coloring agents may also be added. In addition, preservatives may also be added. Suitable examples of pharmaceutically acceptable preservatives includevarious antibacterial and antifungal agents such as solvents, for example ethanol, propylene glycol, benzyl alcohol, chlorobutanol, quaternary ammonium salts, and parabens (such as methyl paraben, ethyl paraben, propyl paraben, etc.).

"Immediate-release" is meant to include a conventional release, in which release of the drug starts immediately after administration. As used herein, the term "immediate release" includes dosage forms that allow the drug to dissolve in the gastrointestinal contents, with no intention of delaying or prolonging the dissolution or absorption of the drug. The objective is for the drug to be released rapidly after administration, for example for it to be possible to release at least 80% of the drug within approximately 30 minutes after commencement of dissolution in a dissolution test.

"Sustained-release" or "extended-release" includes dosage forms whose drug-release characteristics of time course and/or location are chosen to accomplish therapeutical or convenience objectives not offered by conventional dosage forms such as a solution or an immediate release dosage form.

The term "steady-state" means that a plasma level for a given active agent or combination of active agents, has been achieved and which is maintained with subsequent doses of the active agent(s) at a level which is at or above the minimum effective therapeutic level and is below the minimum toxic plasma level for a given active agent(s).

The term "single formulation" as used herein refers to a single carrier or vehicle formulated to deliver effective amounts of both therapeutic agents to a patient. The single vehicle is designed to deliver an effective amount of each of the agents along with any pharmaceutically acceptable carriers or excipients. The vehicle can be a tablet, capsule, pill, or a patch.

The term "unit dose" is used herein to mean simultaneous administration of both agents together, in one dosage form, to the patient being treated. The unit dosecan be a single formulation. In certain embodiments, the unit dose may include one or more vehicles such that each vehicle includes an effective amount of at least one of the agents (Compound 1 or a CDK 4/6 inhibitor) along with pharmaceutically acceptable carriers and excipients. Alternatively, the unit dose is one or more tablets, capsules, pills, or patches administered to the patient at the same time.

The term "dose range" as used herein refers to an upper and a lower limit of an acceptable variation of the amount of agent specified. Typically, a dose of an agent in any amount within the specified range can be administered to patients undergoing treatment.

The term "treat" is used herein to mean to relieve, reduce or alleviate at least one symptom of a disease in a subject. For example, in relation to HCC, the term "treat" may mean to arrest, delay the onset (i.e., the period prior to clinical manifestation of a disease or symptom of a disease) and/or reduce the risk of developing or worsening a symptom of a disease. The term "protect" is used herein to mean prevent delay or treat, or all, as appropriate, development or continuance or aggravation of symptoms of the disease in a subject.

The term "subject" or "patient" is intended to include animals, which are capable of suffering from or afflicted with HCC or IHCC. Examples of subjects or patients include mammals, e.g., humans, dogs, cows, horses, pigs, sheep, goats, cats, mice, rabbits, rats, and transgenic non-human animals. In certain embodiments, the subject is a human, e.g., a human suffering from, at risk of suffering from, or potentially capable of suffering from HCC or IHCC.

The term "about" or "approximately" usually means within 20%, more preferably within 10%, and most preferably still within 5% of a given value or range. Alternatively, especially in biological systems, the term "about" means approximately within a log (i.e., an order of magnitude) preferably within a factor of two of a given value.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, ") unless otherwise noted. Recitation of ranges of values herein are merely intended to serve as a shorthand method of referring individually to each separate value falling within the range, unless otherwise indicated herein, and each separate value is incorporated into the specification as if it were individually recited herein.

The term "enhanced effect" as used herein, refers to action of two agents that administered together provide a greater or improved result than when the individual agents are administered alone without co-administration of the other agent. Administration of the agents together may provide an enhanced effect when they are administered simultaneously or sequentially. Sequential administration of the agents includes administrations separated by several seconds, minutes, hours or days. Administration of the agents together may provide an enhanced effect when the agents are administered either as part of a single formulation, or when administered in separate formulations. Examples of agents that may be administered together include Compound 1 and CDK4/6 inhibtors. Additional examples of agents that may be administered together include i) Compound 1 and ribociclib; ii) Compound 1 and palbociclib; and iii) Compound 1 and abemaciclib.

The enhanced effect's greater or improved result may include, for example, one or more of the following: i) improved quality of tumor response, ii) improved speed of the tumor response, and iii) a tumor response that is more than additive of the response that might otherwise be achieved had the individual agents been administered alone. Examples of improved quality of tumor response may include complete regregression (CR) instead of partial regression (PR), stable disease (SD) or progressive disease (PD). Another example of improved quality of tumor response may

include partial regression (PR) instead of stable disease (SD) or progressive disease (PD). Another example of improved quality of tumor response may include stable disease (SD) instead of progressive disease (PD). Controlled studies to determine whether administration of the agents together resulted with an enhanced effect of a tumor response more than additive of the corresponding responses achieved when the individual agents are respectively administered alone may be done, for example, in mice, rats, dogs, monkeys or other animals. Such controlled studies may evaluate, for example, the resulting tumor volume or metastatic or other status. Likewise, controlled studies may be used to determine an enhanced effect resulting in a faster tumor response.

Furthermore, the treatment may be provided to a subject having hepatocellular carcinoma with altered FGFR4 and /or FGF19 (fibroblast growth factor 19) status.

Alternatively, treatment may include or be performed in conjunction with analyzing FGFR4 and/or FGF19 status in a biological sample containing cells of said hepatocellular carcinoma, and if said hepatocellular carcinoma exhibits an FGFR4 and/or FGF19 alteration, treating a subject with a treatment effective amount of a therapeutic combination as described herein.

Combinations for use

Provided herein is a combination therapy useful for the treatment of HCC or IHCC. As discussed below, combinations provided herein may have a number of advantages.

One advantage of the combination disclosed herein is the unexpected enhanced effect of a combination of Compound 1 and a CDK 4/6 inhibitor on treatment of tumor grown inhibition and treatment of HCC or IHCC.

In some embodiments provided herein is a single pharmaceutical formulation containing a combination of Compound 1 and a CDK 4/6 inhibitor. An advantage provided herein is the enhanced effect that results in the treatment of HCC compared to treatment with a single dose of either drug. When the drugs are provided in a single unit dose or single formulation, the "pill burden" on a patient suffering from HCC is not increased.

As specified above, in one aspect, provided herein is a drug combination for use in treating, preventing, arresting, delaying the onset of and/or reducing the risk of developing, or reversing HCC in a mammal comprising administering to said mammal a combination therapy, comprising an effective amount of Compound 1 and an effective amount of a CDK 4/6 inhibitor.

The subject to be treated (e.g., patient) may be determined to be non-responsive or resistant to one or more HCC therapies, e.g., Compound 1, or the individual to be treated is responsive to Compound 1 therapy, but the therapy was improved with the administration of a CDK 4/6 inhibitor. For example, the patient is administered Compound 1 (e.g., 50 mg to 600 mg per day, 200 mg to 400 mg per day, or 300 mg per day for some period of time, e.g., more than one day, more than two days, more than three days, more than one week, for 21 days, more than one month, etc. After that time, a CDK 4/6 inhibitor could be administered to that patient in combination with Compound 1.

Amounts of CDK 4/6 inhibitor may vary depending on the CDK 4/6 inhibitor that is used. For example, palbociclib may be administered, for example in a dosage of 75, 100, or 125 mg/day; ribociclib may be administered, for example, in a dosage of 200, 400, or 600 mg/day. Typically, a dosage is administered orally as a single capsule for 21 consecutive days followed by a 7 day off-treatment period.

The daily dosage may be part of a cyclic regimen lasting 14 to 21 days or longer. The daily dosage amount may be administered as a single dosage or as multiple dosages.

One skilled in the art appreciates that the effective dose of the active drug may be lower than the actual amount administered. As such, provided herein are doses necessary to achieve a therapeutic dose.

In various embodiments, provided herein are methods of treating HCC by administering an effective amount of Compound 1 and a CDK 4/6 inhibitor, to an individual having HCC. The amount of the combination of agents is effective to treat the HCC. In one embodiment, the combination of agents has an enhanced effect. In one embodiment, even though one or more of the agents administered alone at a particular dosage may be effective, when administered in combination, at the same dosage of each agent, the treatment is more effective. For example, in one embodiment a combination of Compound 1 and palbociclib is more effective than is administration of either agent alone. In another embodiment a combination of Compound 1 and ribociclib is more effective than is administration of either agent alone.

Dosages

The optimal dose of the combination of agents for use in the treatment of HCC can be determined empirically for each individual using known methods and will depend upon a variety of factors, including the activity of the agents; the age, body weight, general health, gender and diet of the individual; the time and route of administration; and other medications the individual is taking. Optimal dosages may be established using routine testing and procedures that are well known in the art.

For the combination therapy of the instant invention, the daily dose of Compound 1 is in the range of 50 mg to

600 mg. Thus, the daily dose of Compound 1 can be up to 600 mg, or, the daily dose of Compound 1 can be up to 400 mg, up to 300 mg,. 200 mg to 400 mg, or, can be 300 mg.

The time of administration can be chosen such that both the drugs are administered simultaneously, separately or sequentially, either in the morning or at night. Alternatively, one drug can be administered in the morning and the other at night, Both of he drugs can be administered as a single tablet, capsule, pill, patch or jelly formulation, once daily, either in the morning or at night.

The amount of combination of agents that may be combined with the carrier materials to produce a single dosage form will vary depending upon the individual treated and the particular mode of administration. The unit dosage forms containing the combination of agents as described herein will contain the amounts of each agent of the combination that are typically administered when the agents are administered alone.

Pharmaceutical Formulations and Routes of Administration

Provided herein are pharmaceutical formulations comprising a combination of agents for the treatment of HCC as set out in the appended set of claims. The pharmaceutical formulations may additionally comprise a carrier or excipient, stabilizer, flavoring agent, and/or coloring agent.

A combination of agents may be administered using a variety of routes of administration known to those skilled in the art. Routes of administration include oral administration. For example, a pharmaceutical formulation comprising a combination of agents may be taken orally in the form of liquid, syrup, tablet, capsule, powder, sprinkle, chewtab, or dissolvable disk. Alternatively, pharmaceutical formulations of the present invention can be administered intravenously or transdermally. Additional routes of administration are known to those skilled in the art (see, e.g., Remington's Pharmaceutical Sciences, Gennaro A. R., Ed., 20. sup.th Edition, Mack Publishing Co., Easton, Pa.).

The Compound 1 and CDK 4/6 inhibitor may be formulated as a paste, jelly, or suspension. For example, the drugs can be dissolved, entrapped or suspended in the form of drug particles, microencapsulated particles, or drug-polymer particles in a gelatinous solution or semi-solid. An advantage of an oral jelly formulation is that it is easier to administer the drugs to patients who have difficulty swallowing tablets, capsules or pills. Both agents can be thoroughly mixed and suspended in an appropriate medium to form a paste or a gel. Additional agents can optionally be mixed to provide flavor during oral administration. Peanut butter or alginate, flavored with raspberry and a sweetener are examples of the many suitable taste masking agents. Alternatively, the paste or jelly can also be formulated with suitable binders or excipients known in the art for topical administration.

Methods of preparing sustained release formulations in the form of tablets, capsules or pills are known in the art.For example, the sustained release formulation is prepared by coating the active ingredient of the drug with a polymer, preferably a water-insoluble polymer. For example, a water-insoluble polymer used in the pharmaceutical field as a sustained release coating agent, an enteric coating agent, or a gastric coating agent. The water-insoluble polymer can include, for example, ethyl cellulose, purified shellac, white shellac, aminoalkyl methacrylate copolymer RS, hydroxypropyl methylcellulose phthalate, hydroxypropyl methylcellulose acetate succinate, carboxymethylethyl-cellulose, cellulose acetate phthalate, methacrylic acid copolymer L, methacrylic acid copolymer LD, methacrylic acid copolymer S, aminoalkyl methacrylate copolymer E, or polyvinyl acetal diethylaminoacetate.

The type, degree of substitution and molecular weight of the water-insoluble polymers can depend on solubility of the active ingredient in water or an alcoholor the desired sustained release level. The water-insoluble polymers can be used either alone or in combination. There can be further incorporated a hydrogenated oil, stearic acid, or cetanol as a coating auxiliary agent, and a middle-chain triglyceride, triacetin, triethyl citrate, or cetanol as a plasticizer.

Furthermore, the sustained release formulation can be a matrix-type tablet or granule. The active ingredient can be coated with up to 3 different types of polymers. These three different types of polymers can include: 1) a water insoluble polymer, such as ethylcellulose; 2) a pH independent gelling polymer, such as hydroxypropyl methylcellulose; and 3) a pH dependent gelling polymer, such as sodium alginate. These three different types of polymers can be used together to attenuate the release rate of the drugs.

Dosage Forms: Release Properties

Sustained-release formulations can achieve a degree of sustained effect. However, the exposure and/or the bioavailability of the active ingredient may vary based on a variety of factors, such as for example, the absorption window, the carriers or excipients used in the formulation, the mode of delivery of the formulation, and/or the transit time of the active ingredient through the gastrointestinal tract of the patient.

A combination therapy can contain at least one sustained-release portion for performing a sustained-release function and one immediate release portion for performing an immediate release function. When the combination therapy is in a single dosage form, it can be in the form of tablets formed from a mixture of sustained-release granules constituting a sustained-release portion and immediate-release granules constituting an immediate-release portion, a capsule preparation obtained by filling a capsule with sustained-release granules and immediate-release granules, or press-coated tablets in which an outer layer constituting an immediate-release portion is formed on an inner core constituting a sustained-release portion. There is, however, no limitation to the above embodiments.

Moreover, there are no particular limitations on the state of containment of each drug in the composition or in an immediate-release portion or a sustained-release portion; the Compound 1 may be dispersed uniformly in the composition, immediate release portion or sustained release portion, or may be contained in only one part of the composition, immediate-release portion or sustained-release portion, or may be contained such that there is a concentration gradient.

A sustained-release portion in the composition according to the present invention can contain at least one non-pH-dependent polymeric substance or pH-dependent polymeric substance for controlling drug release.

A non-pH-dependent polymeric substance used herein can comprise a polymeric substance whose charge state hardly changes under pH conditions generally found in the gastrointestinal tract, specifically from pH 1 to pH 8. This means, for example, a polymeric substance that does not have functional groups whose charge state changes depending on the pH such as basic functional groups such as amino groups or acidic functional groups such as carboxylic acid groups. Note that the non-pH-dependent polymeric substance can be included for giving the composition according to the present invention a sustained-release function but may also be included for another purpose. Moreover, the non-pH-dependent polymeric substance used in the present invention may be water-insoluble, or may swell in water or dissolve in water to form a gel.

Examples of water-insoluble non-pH-dependent polymeric substances includecellulose ethers, cellulose esters, and methacrylic acid-acrylic acid copolymers (trade name Eudragit, manufactured by Rohm GmbH & Co. KG, Darmstadt, Germany). Examples includecellulose alkyl ethers such as ethylcellulose (trade name Ethocel, manufactured by Dow Chemical Company, USA), ethyl methylcellulose, ethyl propylcellulose or isopropylcellulose, and butylcellulose, cellulose aralkyl ethers such as benzyl cellulose, cellulose cyanoalkyl ethers such as cyanoethylcellulose, cellulose organic acid esters such as cellulose acetate butyrate, cellulose acetate, cellulose propionate or cellulose butyrate, and cellulose acetate propionate, ethyl acrylate-methyl methacrylate copolymers (trade name Eudragit NE, manufactured by Rohm GmbH & Co. KG, Darmstadt, Germany), and aminoalkyl methacrylate copolymer RS (trade names Eudragit RL, Eudragit RS). There are no particular limitations on the mean particle diameter of a water-insoluble polymer used in the present invention, but usually the lower this mean particle diameter the better the performance, with the mean particle diameter preferably being from 0.1 to 100 μm, more preferably from 1 to 50 μm, particularly preferably from 3 to 15 μm, most preferably from 5 to 15 μm. Moreover, examples of water-soluble or water-swelling non-pH-dependent polymeric substances includepolyethylene oxide (trade name Polyox, manufactured by Dow Chemical Company, molecular weight 100,000 to 7,000,000), low-substituted hydroxypropyl cellulose (trade name L-HPC, manufactured by Shin-Etsu Chemical, Japan), hydroxypropyl cellulose (trade name HPC, manufactured by Nippon Soda, Co., Ltd, Japan), hydroxypropyl methylcellulose (trade names Metolose 60SH, 65SH, 90SH, manufactured by Shin-Etsu Chemical, Japan), and methylcellulose (trade name Metolose SM, manufactured by Shin-Etsu Chemical, Japan).

Asingle non-pH-dependent polymeric substance may be contained in the composition, or a plurality of the non-pH-dependent polymeric substances may be contained. The non-pH-dependent polymeric substance, if used in embodiments reported herein, may be a water-insoluble polymeric substance, more preferably ethylcellulose, an ethyl acrylate-methyl methacrylate copolymer (trade name Eudragit NE), or an aminoalkyl methacrylate copolymer RS (trade name Eudragit RL, Eudragit RS). Particularly preferable is at least one of ethylcellulose and an aminoalkyl methacrylate copolymer RS. Most preferable is ethylcellulose. There are no particular limitations on the amount of the non-pH-dependent polymeric substance contained in the composition; this amount can be adjusted as appropriate in accordance with the purpose such as controlling sustained drug release.

A pH-dependent polymeric substance that can be used in embodiments reported herein may be a polymeric substance whose charge state changes under pH conditions generally found in the gastrointestinal tract, specifically from pH 1 to pH 8. This means, for example, a polymeric substance having functional groups whose charge state changes depending on the pH such as basic functional groups such as amino groups or acidic functional groups such as carboxylic acid groups. The pH-dependent functional groups of the pH-dependent polymeric substance are preferably acidic functional groups, with the pH-dependent polymeric substance most preferably having carboxylic acid groups.

A pH-dependent polymeric substance used in the present invention may be water-insoluble, or may swell in water or dissolve in water to form a gel. Examples of pH-dependent polymeric substances used in the present invention include, but are not limited to, enteric polymeric substances. Examples of enteric polymeric substances includemethacrylic acid-methyl methacrylate copolymers (Eudragit L100, Eudragit S 100, manufactured by Rohm GmbH & Co. KG, Darmstadt, Germany), methacrylic acid-ethyl acrylate copolymers (EudragitL100-55, Eudragit L30D-55, manufactured by Rohm GmbH & Co. KG, Darmstadt, Germany), hydroxypropyl methylcellulose phthalate (HP-55, HP-50, manufactured by Shin-Etsu Chemical, Japan), hydroxypropyl methylcellulose acetate succinate (AQOAT, manufactured by Shin-Etsu Chemical, Japan), carboxymethyl ethylcellulose (CMEC, manufactured by Freund Corporation, Japan), and cellulose acetate phthalate.

Examples of pH-dependent polymeric substances that swell in water or dissolve in water to form a gel includealginic acid, pectin, carboxyvinyl polymer, and carboxymethyl cellulose. In the present invention, a single pH-dependent polymeric substance may be contained in the composition, or a plurality of pH-dependent polymeric substances may be contained. The pH-dependent polymeric substance used in the present invention is preferably an enteric polymeric substance, more preferably a methacrylic acid-ethyl acrylate copolymer, a methacrylic acid-methyl methacrylate copolymer, hydroxypropyl methylcellulose phthalate, or hydroxypropyl methylcellulose acetate succinate, particularly preferably a methacrylic acid-ethyl acrylate copolymer.

When using a pH-dependent polymeric substance in the manufacturing process of a composition according to the present invention, a commercially available product of a powder type or a granular type, or a suspension type in which the pH-dependent polymeric substance has been dispersed in a solvent in advance can be used as is, or such a commercially available product can be used dispersed in water or an organic solvent. The lower the particle diameter of the pH-dependent polymeric substance the better the performance, with the pH-dependent polymeric substance preferably being of the powder type. In the case of a methacrylic acid-ethyl acrylate copolymer, an example is Eudragit L100-55. There are no particular limitations on the mean particle diameter of a pH-dependent polymeric substance used in the present invention, but the mean particle diameter is preferably from 0.05 to 100 $\mu$m, more preferably from 0.05 to 70 $\mu$m, most preferably from 0.05 to 50 $\mu$m. Moreover, there are no particular limitations on the amount of the pH-dependent polymeric substance, for example, in the case of an enteric polymeric substance, the amount is generally from 0.1 to 90 parts by weight, preferably from 1 to 70 parts by weight, more preferably from 5 to 60 parts by weight, particularly preferably from 10 to 50 parts by weight, based on 100 parts by weight of the composition.

A combination therapy according to embodiments reported herein may further contain any of various additives, such as any of various pharmacologically acceptable carriers such as diluents, lubricants, binders and disintegrants, as well as preservatives, colorants, sweeteners, plasticizers, film coating agents as necessary. Examples of diluents includelactose, mannitol, dibasic calcium phosphate, starch, pregelatinized starch, crystalline cellulose, light silicic anhydride, synthetic aluminum silicate, magnesium aluminate metasilicate. Examples of lubricants includemagnesium stearate, calcium stearate, talc, sodium stearyl fumarate. Examples of binders includehydroxypropyl cellulose, methylcellulose, sodium carboxymethyl cellulose, hydroxypropyl methylcellulose, polyvinylpyrrolidone. Examples of disintegrants includecarboxymethyl cellulose, calcium carboxymethyl cellulose, croscarmellose sodium, sodium carboxymethyl starch, low-substituted hydroxypropyl cellulose. Examples of preservatives includeparaoxybenzoic acid esters, chlorobutanol, benzyl alcohol, phenethyl alcohol, dehydroacetic acid, sorbic acid. Preferable examples of colorants include, water-insoluble lake pigments, natural pigments (e.g., beta-carotene, chlorophyll, red ferric oxide), yellow ferric oxide, red ferric oxide, black ferric oxide. Preferable examples of sweeteners includesodium saccharin, dipotassium glycyrrhizate, aspartame, stevia. Examples of plasticizers includeglycerol fatty acid esters, triethyl citrate, propylene glycol, polyethylene glycol. Examples of film coating agents includehydroxypropyl methylcellulose, hydroxypropyl cellulose.

## Manufacturing Methods

To manufacture embodiments as reported herein, a single conventional method, or a combination of conventional methods, can be used. For example, when manufacturing drug-containing granules as a sustained-release portion or an immediate-release portion, granulation is the main operation, but this may be combined with other operations such as mixing, drying, sieving, and classification. As the granulation method, for example, a wet granulation method in which a binder and a solvent are added to the powder and granulation is carried out, a dry granulation method in which the powder is compressed and granulation is carried out, a molten granulation method in which a binder that melts on heating is added and heating and granulation are carried out can be used.

Furthermore, in accordance with the granulation method, an operating method such as a mixing granulation method using a planetary mixer, a screw mixer, a high-speed mixing granulation method using a Henschel mixer, a Super mixer, an extruding granulation method using a cylindrical granulator, a rotary granulator, a screw extruding granulator, a pellet mill type granulator, a wet high-shear granulation method, a fluidized-bed granulation method, a compression granulation method, a crushing granulation method, or a spraying granulation method can be used. After the granulation, drying using a dryer, a fluidized bed, cracking, and sieving can be carried out to obtain the granules or fine granules for use. Moreover, a granulation solvent may be used when preparing the composition according to the present invention. There are no particular limitations on such a granulation solvent, which may be water or any of various organic solvents, for example, water, a lower alcohol such as methanol or ethanol, a ketone such as acetone or methyl ethyl ketone, methylene chloride, or a mixture thereof.

For sustained-release granules contained in embodiments, at least one drug and at least one selected from non-pH-dependent polymeric substances and pH-dependent polymeric substances are mixed together, a diluent and a binder are added as necessary, and granulation is carried out to obtain granular matter. The granular matter obtained is dried using a tray dryer, a fluidized bed dryer, and sieving is carried out using a mill or an oscillator, whereby the sustained-release granules can be obtained. Alternatively, as a method of manufacturing sustained-release granules in the present invention, it is possible to add at least one drug, at least one selected from non-pH-dependent polymeric

substances and pH-dependent polymeric substances, and as necessary a diluent and a binder using a dry compactor such as a roller compactor or a slug tabletting machine, and carry out compression-molding while mixing, and then carry out granulation by cracking down to a suitable size. The granular matter prepared using such a granulator may be used as is as granules or fine granules according to the present invention, or may be further cracked using a power mill, a roll granulator, a rotor speed mill, and sieved to obtain sustained-release granules. Note that immediate-release granules can also be manufactured as for the sustained-release granules.

A compression-molded product can be manufactured as a drug-containing sustained-release portion or immediate-release portion, or as a composition reported herein using a single conventional method, or a combination of conventional methods. For example, at least one drug, at least one selected from non-pH-dependent polymeric substances and pH-dependent polymeric substances, a diluent such as mannitol or lactose, a binder such as polyvinylpyrrolidone or crystalline cellulose, a disintegrant such as carmellose sodium or crospovidone, and a lubricant such as magnesium stearate or talc are used, and tableting is carried out using an ordinary method, whereby the compression-molded product can be obtained. In this case, tabletting is the main operation in the method of manufacturing the compression-molded product, but this may be combined with other operations such as mixing, drying, sugar coating formation, and coating.

Examples of the method for the tabletting includedirect compression molding in which at least one drug and pharmacologically acceptable additives are mixed together and then the mixture is directly compression-molded into tablets using a tabletting machine, and dry granule compression or wet granule compression in which sustained-release granules or immediate-release granules according to the present invention are subjected to compression-molding after adding a lubricant or a disintegrant as necessary. There are no particular limitations on the tabletting machine used in the compression molding; for example, a single-punch tabletting machine, a rotary tabletting machine, or a press-coated tabletting machine can be used.

Drug-containing sustained-release granules or immediate-release granules, or compression-molded product according to embodiments herein can be used as is in the form of granules or a tablet as the composition, but may also be subjected to further processing to manufacture the composition. For example, the compression-molded product or granules can be given a film coating using a film base material such as ethylcellulose, casein, methylcellulose, hydroxypropyl methylcellulose, methacrylic acid copolymer L, cellulose acetate phthalate, shellac, or given a sugar coating using a sugar coating liquid containing saccharose, sugar alcohol, gum arabic powder, talc, thus producing film-coated tablets or sugar-coated tablets. One solvent in this coating technique may be purified water, but an organic solvent such as an alcohol, a ketone, an ether or a chlorinated hydrocarbon, or a mixture thereof can also be used. For example, ethanol, acetone, methylene chloride can be used as an organic solvent. Moreover, as the coating apparatus, an apparatus ordinarily used in coating techniques for manufacturing medicines can be used, with examples including a spray coating apparatus in which the coating is carried out by spraying a coating liquid, and a rotor fluidized bed granulator for layering.

In the case of manufacturing capsule preparations, capsule preparations can be manufactured by filling sustained-release granules or immediate-release granules as above, or mini-tablets into hard gelatin capsules or HPMC capsules using an automatic capsule filling machine. Alternatively, in the case of the preparations for per-tube administration or a dry syrup that is used mixed with water when taken, sustained-release granules or immediate-release granules as above can be mixed with a thickener or a dispersant so as to disperse these granules, the mixture then being made into granules or tablets. Furthermore, a liquid or jelly can be made using water, and substances selected from dispersants, emulsifiers, thickeners, preservatives, pH adjustors, sweeteners, flavorings, fragrances. However, with respect to other manufacturing methods, there are no limitations to the above.

So that embodiments described herein may be more fully understood, the following examples are set forth.

## EXAMPLES

### Materials and methods

#### Cell Lines Tested

The cell line used, JHH7, was sourced from Japanese Collection of Research Bioresources Cell Bank (JCRB), verified free of mycobacterium contamination and verified for identity by short tandem repeat analysis of 9 markers.

#### Cell Line Maintenance and Study Conditions

JHH7 cells were maintained in William's E Medium (Thermo Fisher Scientific, 12551-032) with 10% fetal bovine serum. Cells were maintained prior to and during experiments at 37 °C, 5% $CO_2$, and at 95% relative humidity. Cell passage number was limited to between 12 and 20.

Xenograft Generation, Dosing and Measurement of Antitumor Activity

The human hepatocellular cancer cell line JHH7 was cultured in William's E Medium (Thermo Fisher Scientific, 12551-032) containing 10% fetal bovine serum at 37 °C in a 5% $CO_2$ atmosphere and kept in exponential growth phase. For harvesting, the cells were washed with phosphate buffered saline, incubated with 0.25% trypsin-EDTA, and suspended in a 1:1 mixture of William's E Medium and Matrigel (Coming) at a final concentration of $5 \times 10^7$cells/mL. To generate xenografts, 0.1-mL of the inoculum was injected subcutaneously into the right flank region of NU/NU immuno compromised 6-8 week old female mice, giving a final concentration of $5 \times 10^6$ cells/mouse. When the mean tumor volume (TV) reached approximately 170 $mm^3$ (10 days after implantation), 144 mice were selected based on their TVs, and were sorted into 18 treatment groups with 8 animals per group. Per os (PO) treatment with Compound 1 (300 and 500 mg/kg) alone or in combination with vehicle (control) or palbociclib (50 and 100mg/kg) or ribociclib (75 and 150mg/kg) administered once daily (QD) continued for 8 days. The administration volume (0.1 mL/10 g body weight) was calculated from the individual mouse body weight (BW) before administration. Body weights were measured daily and tumor measurements were performed twice weekly.

The human primary hepatocellular carcinoma model LIX066 model from ChemPartner (shangpharma.com) were implanted into the female severe combined immunodeficiency (SCID) mice. When the tumors developed in mice, the mice were sacrificed and the tumors were resected and implanted into female nude mice for tumor preservation, histopathology diagnosis and in vivo efficacy study. Solid tumor tissues were depleted of necrotic components, cut into 10-15 mg pieces, and mixed. Three to five pieces were mixed with 15-30 mL Matrigel and implanted into a flank of 6-8-week-old female NU/NU immune compromised mice weighing 18-20 g nude mice. 30 mice with average tumor volume of 160 $mm^3$ selected and randomized into 6 groups of 5 mice each. All of the primary human tumors utilized in this study had undergone 3-5 passages in vivo and the tumor histology of each was maintained over the serial transplantation process. Per os (PO) treatment with Compound 1 (300 and 500 mg/kg) alone or in combination with vehicle (control) or palbociclib (100mg/kg) administered once daily (QD) continued for 17 days. The administration volume (0.1 mL/10 g body weight) was calculated from the individual mouse body weight (BW) before administration. Body weights were measured daily and tumor measurements were performed twice weekly.

The TV in $mm^3$ was calculated according to the following formula: TV = length $\times$ width$^2$ $\times$ 0.5 length: largest diameter of tumor (mm) width: diameter perpendicular to length (mm) The Tumor Growth Inhibition% (TGI) was calculated according to the following formula:

$$\frac{\text{Average Control TV Day X} - \text{Treatment TV Day X}}{\text{Average Control TV Day X}} \times 100$$

where Day X is any day of measurement

The anti-tumor effects of the treatment, Partial (PR) and complete regression (CR), stable (SD) and progressive (PD) disease were defined by the Xenograft Model Response Criteria (see below). Mice with > 20% body weight loss compared to their Day 1 body weight or bearing tumors with the longest diameter > 2000 mm were immediately euthanized to prevent any pain or suffering in the animal as according to IACUC guidelines.

Statistical Analysis

Data are expressed as the mean $\pm$SEM for Tumor Volume (TV). The differences in TV between groups were analyzed by two way ANOVA followed by Sidak post hoc test. Statistical analyses were performed using the GraphPad Prism version 6 (GraphPad Software, La Jolla, CA).

Xenograft Model Response Criteria

Progressive disease (PD): 3 consecutive measurements >120% of starting volume or 3 consecutive increasing measurements from best response, Stable disease (SD): 3 consecutive measurements >50% and <120% of starting volume, Partial regression (PR): 3 consecutive measurements <50% of starting volume, Complete regression (CR): 3 consecutive measurements <30$mm^3$.

Formulation of CDK 4/6 Inhibitors

In the examples reported below, ribociclib and palbociclib were formulated as follows. This type of formulation is exemplary and not required in particular embodiments of the invention. In these examples both ribociclib and palbociclib were presented as free bases.

Palbociclib was formulated in a 25mM Sodium Bicarbonate, 15mM Lactic Acid solution with 2% Cremaphor. Add cremaphor first and sonicate until a fine, even suspension is formed. This compound should be made fresh each time.
Ribociclib was formulated in a 0.5% Methylcellulose in distilled water with 1% Cremaphor. Add Cremaphor first and sonicate until a fine even suspension is formed. This compound should be made fresh each time

Example 1 - Compound 1 and Ribociclib

The JHH7 cell line was grown as a xenograft in female nude immunocompromised mice and tumor bearing mice were orally treated daily for 8 days with 300 or 500mg/kg Compound 1 as single agent or in combination with 75 and 150mg/kg ribociclib. Ribociclib, as single agent at 75mg/kg did not significantly inhibit tumor growth with 13% TGI whereas 150mg/kg significantly inhibit tumor growth ($P \leq 0.0001$) with 23% TGI in comparison to the vehicle controls. The single agent Compound 1 at 300 and 500mg/kg resulted in significant inhibition of tumor growth ($P \leq 0.0001$) with TGI of 14% and 35%, respectively in comparison to the vehicle controls. The combination of 300mg/kg Compound 1 and 75mg/kg ribociclib resulted in significant enhancement of tumor growth inhibition ($P \leq 0.0001$) in comparison to 300mg/kg Compound 1 single agent with TGI of 44%. The combination of 300mg/kg Compound 1 and 150mg/kg ribociclib also resulted in significant enhancement of tumor growth inhibition ($P \leq 0.0001$) in comparison to the 300mg/kg Compound 1 alone with TGI of 64%. The combination of 500mg/kg Compound 1 and 75mg/kg ribociclib resulted in significant enhancement of tumor growth inhibition ($P \leq 0.0001$ in comparison to 500mg/kg Compound 1 alone with TGI of 61%. The combination of 500mg/kg Compound 1 and 150mg/kg Ribociclib also resulted in significant enhancement of tumor growth inhibition ($P \leq 0.0001$) in comparison to the 500mg/kg Compound 1 alone with TGI of 73%.
These data demonstrate that ribociclib can significantly enhance the antitumor effects of Compounr 1. Treatment with Compound 1 as single agent or in combination with Ribociclib did not cause any CR, PR or SD and all groups had PD. All combination dosing groups were well tolerated based on body weight measurements and routine clinical observation.
Results are shown in FIG. 1A through FIG. 1C.

Example 2 - Compound 1 and Palbociclib

The JHH7 cell line was grown as a xenograft in female nude immunocompromised mice and tumor bearing mice were orally treated daily for 8 days with 300 or 500mg/kg Compound 1 as single agent or in combination with 50 and 100mg/kg palbociclib. Palbociclib, as single agent at 50mg/kg did not significantly inhibit tumor growth with 11% TGI whereas 100mg/kg significantly inhibit tumor growth ($P \leq 0.05$) with 26% TGI in comparison to the vehicle controls. The single agent Compound 1 at 300mg/kg resulted in significant inhibition of tumor growth ($P \leq 0.01$) with TGI of 12% and 500mg/kg also resulted in significant tumor growth inhibition ($P \leq 0.0001$) with 32% in comparison to the vehicle controls. The combination of 300mg/kg Compound 1 and 50mg/kg Palbociclib resulted in significant enhancement of tumor growth inhibition ($P \leq 0.0001$) in comparison to 300mg/kg Compound 1 single agent with TGI of 59%.
The combination of 300mg/kg Compound 1 and 100mg/kg palbociclib also resulted in significant enhancement of tumor growth inhibition ($P \leq 0.0001$) in comparison to the 300mg/kg Compound 1 alone with TGI of 77%. The combination of 500mg/kg Compound 1 and 50mg/kg palbociclib resulted in significant enhancement of tumor growth inhibition ($P \leq 0.0001$) in comparison to 500mg/kg Compound 1 alone with TGI of 62%. The combination of 500mg/kg Compound 1 and 100mg/kg palbociclib also resulted in significant enhancement of tumor growth inhibition ($P \leq 0.0001$) in comparison to the 500mg/kg Compound 1 alone with TGI of 77%.
These data demonstrate that palbociclib can significantly enhance the antitumor effects of Compound 1. Treatment with Compound 1 as single agent or in combination with palbociclib did not cause any CR, PR or SD and all groups had PD. All combination dosing groups were well tolerated based on body weight measurements and routine clinical observation.
Results are shown in FIG. 2A through FIG. 2C.

Example 3 - Compound 1 and Palbociclib

In a patient derived xenograft model (PDX), LIX066 PDX fragments were inoculated in female nude immunocompromised mice and tumor bearing mice were orally treated daily for 17 days with 300 or 500mg/kg Compound 1 as single agent or in combination with 100mg/kg palbociclib. Palbociclib, as single agent at 100mg/kg significantly inhibited tumor growth ($P \leq 0.001$), in comparison to the vehicle controls, with 62% TGI. All the enrolled animals in this group showed PD. The single agent Compound 1 at 300mg/kg and 500mg/kg significantly inhibited tumor growth ($P \leq 0.0001$) in comparison to the vehicle controls, with 59% TGI and 70% TGI, respectively. All animals in the 300mg/kg and the 500mg/kg Compound 1 groups showed PD.
The combination of 300mg/kg Compound 1 and 100mg/kg palbociclib resulted in significant enhancement of

tumor growth inhibition (P ≤ 0.0001) in comparison to 300mg/kg Compound 1 alone with TGI of 96%. This combination led to SD in 3/5 animals and PR in 2/5 animals. The combination of 500mg/kg Compound 1 and 100mg/kg palbociclib also resulted in significant enhancement of tumor growth inhibition (P ≤ 0.0001) in comparison to the 300mg/kg Compound 1 alone with TGI of 97%. This combination led to SD in 1/5 animals and PR in 4/5 animals. All combination dosing groups were well tolerated based on body weight measurements and routine clinical observation.

Results are shown in FIG. 3A and FIG. 3B. Consistent with the discovery that Palbociclib can enhance Compound 1 antitumor effects in HCC models, additional testing in 7 more PDX models showed similar enhancement in 3 out of 7 models.

## Claims

1. A combination of *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide or a pharmaceutically acceptable salt thereof and a CDK 4/6 inhibitor or a pharmaceutically acceptable salt thereof for use in treating hepatocellular carcinoma in a patient in need thereof.

2. The combination for use according to claim 1, wherein the *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide or a pharmaceutically acceptable salt thereof is administered in a daily dosage selected from the group consisting of between 50 mg to 600 mg/day, between 200 mg to 400 mg/day, between 50 mg/day and 3000 mg/day, 150 mg/day, 300 mg/day, 600 mg/day, 1000 mg/day, 1500 mg/day, and 2000 mg/day.

3. The combination for use according to claim 1, wherein the CDK 4/6 inhibitor is selected from the group consisting of 6-acetyl-8-cyclopentyl-5-methyl-2-{[5-(piperazin-1-yl)pyridin-2yl]amino}pyrido[2,3-*d*]pyrimidin-7(8*H*)-one (palbociclib) and pharmaceutically acceptable salts thereof; *N*-(5-((4-ethylpiperazin-1-yl)methyl)pyridin-2-yl)-5-fluoro-4-(4-fluoro-1-isopropyl-2-methyl-*1H*-benzo[d]imidazol-6-yl)pyrimidin-2-amine (abemaciclib) and pharmaceutically acceptable salts thereof; and 7-cyclopentyl-2-(5-piperazin-1-yl-pyridin-2-ylamino)-7H-pyrrolo[2,3-d]pyrimidine-6-carboxylic acid dimethylamide (ribociclib); 2'-((5-(4-isopropylpiperazin-1-yl)pyridin-2-yl)amino)-7', 8'dihydro-6'Hspiro[cyclohexane1,9' pyrazino[1',2':1,5] pyrrolo[2,3-d]pyrimidin]-6'-one (G1T-38); 2'-((5-(4-methylpiperazin-1-yl)pyridin-2-yl)amino)-7',8'-dihydro-6'H-spiro[cyclohexane-1,9'-pyrazino[1',2':1,5]pyrrolo[2,3-d]pyrimidin]-6'-one (G1T-28); N-(4-piperidinyl)-4-(2,6-dichlorobenzoylamino)-1*H*-pyrazole-3 carboxamide (AT-7519); 2-Hydroxy-1-[2-[[9-(trans-4-methylcyclohexyl)-9*H*-pyrido[4',3':4,5]pyrrolo[2,3-d]pyrimidin-2-yl]amino]-7,8-dihydro-1,6-naphthyridin-6(5*H*)-yl]ethanone (FLX-925); 2-(2-chlorophenyl)-5,7-dihydroxy-8-((3*S*,4*R*)-3-hydroxy-1-methylpiperidin-4-yl)-4*H*-chromen-4-one (alvocidib) and pharmaceutically acceptable salts thereof.

4. The combination for use according to claim 1, wherein the CDK 4/6 inhibitor is palbociclib.

5. The combination for use according to claim 4, wherein the palbociclib is administered in a daily dosage selected from the group consisting of 75 mg/day, 100 mg/day, and 125 mg/day.

6. The combination for use according to claim 1, wherein the CDK 4/6 inhibitor is ribociclib.

7. The combination for use according to claim 6, wherein the ribociclib is administered in a daily dosage selecting from the group consisting of 200 mg/day, 400 mg/day, and 600 mg/day.

8. The combination for use according to claim 1, wherein the CDK 4/6 inhibitor is abemaciclib.

9. The combination for use according to claim 8, wherein the abemaciclib is administered in a daily dosage selected from the group consisting of 200 mg/day, 300 mg/day, and 400 mg/day.

10. The combination for use according to claim 1, wherein the *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide or a pharmaceutically acceptable salt thereof and the CDK 4/6 inhibitor or pharmaceutically acceptable salt thereof are administered optionally as separate formulations, or as as a single formulation, or are administered sequentially, or are administered simultaneously.

11. The combination for use according to claim 1, wherein the *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide is the free base form of *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide.

**12.** The combination for use according to claim 11, wherein the *N* (2-((6-(3-(2,6-dichloro-3,5-dimethoxyphenyl)-1-methylureido)pyrimidin-4-yl)amino)-5-(4-ethylpiperazin-1-yl)phenyl)acrylamide or a pharmaceutically acceptable salt thereof is administered as a single dosage or in multiple dosages.

**13.** The combination for use according to claim 1, wherein the pharmaceutically acceptable salt of *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide is a hydrochloride salt form of *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide.

**14.** A pharmaceutical formulation comprising *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide or a pharmaceutically acceptable salt thereof and a CDK 4/6 inhibitor or a pharmaceutically acceptable salt thereof.

**15.** The pharmaceutical formulation of claim 14, comprising a free base form of *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide.

**16.** The pharmaceutical formulation of claim 14, wherein the pharmaceutically acceptable salt of *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide is a hydrochloride salt form of *N*-(2-((6-(3-(2,6-dichloro-3,5-dimethoxyphenyl)-1-methylureido)pyrimidin-4-yl)amino)-5-(4-ethylpiperazin-1-yl)phenyl)acrylamide.

## Patentansprüche

**1.** Kombination von *N*-(2-((6-(3-(2,6-Dichlor-3,5-dimethoxyphenyl)-1-methylureido)pyrimidin-4-yl)-amino)-5-(4-ethylpiperazin-1-yl)phenyl)acrylamid oder einem pharmazeutisch unbedenklichen Salz davon und einem CDK-4/6-Inhibitor oder einem pharmazeutisch unbedenklichen Salz davon zur Verwendung bei der Behandlung von hepatozellulärem Karzinom bei einem Patienten, bei dem diesbezüglicher Bedarf besteht.

**2.** Kombination zur Verwendung nach Anspruch 1, wobei das *N*-(2-((6-(3-(2,6-Dichlor-3,5-dimethoxyphenyl)-1-methylureido)pyrimidin-4-yl)amino)-5-(4-ethylpiperazin-1-yl)phenyl)acrylamid oder ein pharmazeutisch unbedenkliches Salz davon mit einer täglichen Dosierung verabreicht wird, die aus der Gruppe bestehend aus zwischen 50 mg bis 600 mg/Tag, zwischen 200 mg bis 400 mg/Tag, zwischen 50 mg/Tag und 3000 mg/Tag, 150 mg/Tag, 300 mg/Tag, 600 mg/Tag, 1000 mg/Tag, 1500 mg/Tag und 2000 mg/Tag ausgewählt ist.

**3.** Kombination zur Verwendung nach Anspruch 1, wobei der CDK-4/6-Inhibitor aus der Gruppe bestehend aus 6-Acetyl-8-cyclopentyl-5-methyl-2-{[5-(piperazin-1-yl)pyridin-2yl]amino}pyrido[2,3-*d*]pyrimidin-7(8*H*)-on (Palbociclib) und pharmazeutisch unbedenklichen Salzen davon; *N*-(5-((4-Ethylpiperazin-1-yl)methyl)pyridin-2-yl)-5-fluor-4-(4-fluor-1-isopropyl-2-methyl-*1H*-benzo[d]imidazol-6-yl)pyrimidin-2-amin (Abemaciclib) und pharmazeutisch unbedenklichen Salzen davon und 7-Cyclopentyl-2-(5-piperazin-1-yl-pyridin-2-ylamino)-7H-pyrrolo[2,3-d]pyrimidin-6-carbonsäuredimethylamid (Ribociclib); 2'-((5-(4-Isopropylpiperazin-1-yl)pyridin-2-yl)amino)-7',8'-dihydro-6'H-spiro[cyclohexan-1,9'-pyrazino-[1',2':1,5]pyrrolo[2,3-d]pyrimidin]-6'-on (G1T-38); 2'-((5-(4-Methylpiperazin-1-yl)pyridin-2-yl)amino)-7',8'-dihydro-6'H-spiro[cyclohexan-1,9'-pyrazino[1',2':1,5]pyrrolo[2,3-d]pyrimidin]-6'-on (G1T-28); N-(4-Piperidinyl)-4-(2,6- dichlorbenzoyl-amino)-1*H*-pyrazol-3-carboxamid (AT-7519); 2-Hydroxy-1-[2-[[9-(trans-4-methylcyclohexyl)-9*H*-pyrido[4',3':4,5]pyrrolo[2,3-d]pyrimidin-2-yl]-amino]-7,8-dihydro-1,6-naphthyridin-6(5*H*)-yl]-ethanon (FLX-925); 2-(2-Chlorphenyl)-5,7-dihydroxy-8-((3*S*,4*R*)-3-hydroxy-1-methylpiperidin-4-yl)-4*H*-chromen-4-on (Alvocidib) und pharmazeutisch unbedenklichen Salzen davon ausgewählt ist.

**4.** Kombination zur Verwendung nach Anspruch 1, wobei es sich bei dem CDK-4/6-Inhibitor um Palbociclib handelt.

**5.** Kombination zur Verwendung nach Anspruch 4, wobei das Palbociclib in einer täglichen Dosierung verabreicht wird, die aus der Gruppe bestehend aus 75 mg/Tag, 100 mg/Tag und 125 mg/Tag ausgewählt ist.

**6.** Kombination zur Verwendung nach Anspruch 1, wobei es sich bei dem CDK-4/6-Inhibitor um Ribociclib handelt.

**7.** Kombination zur Verwendung nach Anspruch 6, wobei das Ribociclib in einer täglichen Dosierung verabreicht wird, die aus der Gruppe bestehend aus 200 mg/Tag, 400 mg/Tag und 600 mg/Tag ausgewählt ist.

**8.** Kombination zur Verwendung nach Anspruch 1, wobei es sich bei dem CDK-4/6-Inhibitor um Abemaciclib handelt.

**9.** Kombination zur Verwendung nach Anspruch 8, wobei das Abemaciclib in einer täglichen Dosierung verabreicht wird, die aus der Gruppe bestehend aus 200 mg/Tag, 300 mg/Tag und 400 mg/Tag ausgewählt ist.

**10.** Kombination zur Verwendung nach Anspruch 1, wobei das *N*-(2-((6-(3-(2,6-Dichlor-3,5-dimethoxyphenyl)-1-methylureido)pyrimidin-4-yl)amino)-5-(4-ethyl-piperazin-1-yl)phenyl)acrylamid oder ein pharmazeutisch unbedenkliches Salz davon und der CDK-4/6-Inhibitor oder das pharmazeutisch unbedenkliche Salz davon als separate Formulierungen oder als eine einzige Formulierung verabreicht werden oder aufeinanderfolgend verabreicht werden oder gleichzeitig verabreicht werden.

**11.** Kombination zur Verwendung nach Anspruch 1, wobei es sich bei dem *N*-(2-((6-(3-(2,6-Dichlor-3,5-dimethoxyphenyl)-1-methylureido)pyrimidin-4-yl)-amino)-5-(4-ethylpiperazin-1-yl)phenyl)acrylamid um die freie Basenform von *N*-(2-((6-(3-(2,6-Dichlor-3,5-dimethoxyphenyl)-1-methylureido)-pyrimidin-4-yl)amino)-5-(4-ethylpiperazin-1-yl)-phenyl)acrylamid handelt.

**12.** Kombination zur Verwendung nach Anspruch 11, wobei das *N*-(2-((6-(3-(2,6-Dichlor-3,5-dimethoxyphenyl)-1-methylureido)pyrimidin-4-yl)amino)-5-(4-ethyl-piperazin-1-yl)phenyl)acrylamid oder ein pharmazeutisch unbedenkliches Salz davon in Form einer einzigen Dosierung oder mehreren Dosierungen verabreicht wird.

**13.** Kombination zur Verwendung nach Anspruch 1, wobei es sich bei dem pharmazeutisch unbedenklichen Salz von *N*-(2-((6-(3-(2,6-Dichlor-3,5-dimethoxyphenyl)-1-methylureido)pyrimidin-4-yl)amino)-5-(4-ethylpiperazin-1-yl)phenyl)acrylamid um eine Hydrochloridsalzform von *N*-(2-((6-(3-(2,6-Dichlor-3,5-dimethoxyphenyl)-1-methylureido)pyrimidin-4-yl)amino)-5-(4-ethylpiperazin-1-yl)-phenyl)acrylamid handelt.

**14.** Pharmazeutische Formulierung, umfassend *N*-(2-((6-(3-(2,6-Dichlor-3,5-dimethoxyphenyl)-1-methyl-ureido)pyrimidin-4-yl)amino)-5-(4-ethylpiperazin-1-yl)phenyl)acrylamid oder ein pharmazeutisch unbedenkliches Salz davon und einen CDK-4/6-Inhibitor oder ein pharmazeutisch unbedenkliches Salz davon.

**15.** Pharmazeutische Formulierung nach Anspruch 14, umfassend eine freie Basenform von *N*-(2-((6-(3-(2,6-Dichlor-3,5-dimethoxyphenyl)-1-methylureido)-pyrimidin-4-yl)amino)-5-(4-ethylpiperazin-1-yl)-phenyl)acrylamid.

**16.** Pharmazeutische Formulierung nach Anspruch 14, wobei es sich bei dem pharmazeutisch unbedenklichen Salz von *N*-(2-((6-(3-(2,6-Dichlor-3,5-dimethoxyphenyl)-1-methylureido)pyrimidin-4-yl)amino)-5-(4-ethylpiperazin-1-yl)phenyl)acrylamid um eine Hydrochloridsalzform von *N*-(2-((6-(3-(2,6-Dichlor-3,5-dimethoxyphenyl)-1-methylureido)pyrimidin-4-yl)-amino)-5-(4-ethylpiperazin-1-yl)phenyl)acrylamid handelt.

## Revendications

**1.** Combinaison de *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide ou d'un sel pharmaceutiquement acceptable correspondant et d'un inhibiteur de CDK 4/6 ou d'un sel pharmaceutiquement acceptable correspondant pour une utilisation dans le traitement d'un carcinome hépatocellulaire chez un patient qui en a besoin.

**2.** Combinaison pour une utilisation selon la revendication 1, le *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide ou un sel pharmaceutiquement acceptable correspondant étant administré en un dosage quotidien choisi dans le groupe constitué par entre 50 mg et 600 mg/jour, entre 200 mg et 400 mg/jour, entre 50 mg/jour et 3 000 mg/jour, 150 mg/jour, 300 mg/jour, 600 mg/jour, 1 000 mg/jour, 1 500 mg/jour, et 2 000 mg/jour.

**3.** Combinaison pour une utilisation selon la revendication 1, l'inhibiteur de CDK 4/6 étant choisi dans le groupe constitué par 6-acétyl-8-cyclopentyl-5-méthyl-2-{ [5-(pipérazin-1-yl)pyridin-2yl]amino}pyrido[2,3-*d*]pyrimidin-7(8*H*)-one (palbociclib) et des sels pharmaceutiquement acceptables correspondants ; *N*-(5-((4-éthylpipérazin-1-yl)méthyl)pyridin-2-yl)-5-fluoro-4-(4-fluoro-1-isopropyl-2-méthyl-*1H*-benzo[d]imidazol-6-yl)pyrimidin-2-amine (abémaciclib) et des sels pharmaceutiquement acceptables correspondants ; et acide 7-cyclopentyl-2-(5-pipérazin-1-yl-pyridin-2-ylamino)-7H-pyrrolo[2,3-d]pyrimidine-6-carboxylique diméthylamide (ribociclib) ; 2'-((5-(4-isopropylpipérazin-1-yl)pyridin-2-yl)amino)-7', 8'dihydro-6'Hspiro[cyclohexane1,9'pyrazino[1',2':1,5]pyrrolo [2,3-d]pyrimidin]-6'-one (G1T-38) ; 2'-((5-(4-méthylpipérazin-1-yl)pyridin-2-yl)amino)-7',8'-dihydro-6'H-spiro[cyclohexane-1,9'-pyrazino[1',2':1,5]pyrrolo[2,3-d]pyrimidin]-6'-one (G1T-28) ; N-(4-pipéridinyl)-4-(2,6-dichlorobenzoylamino)-1*H*-pyrazole-3carboxamide (AT-7519) ; 2-Hydroxy-1-[2-[[9-(trans-4-méthylcyclohexyl)-9*H*-pyrido[4',3':4,5]pyrrolo[2,3-d]pyrimidin-2-yl]amino]-7,8-dihydro-1,6-naphthyridin-6(5*H*)-yl]éthanone (FLX-925) ; 2-(2-chlorophényl)-5,7-dihydroxy-8-((3*S*,4*R*)-3-hydroxy-1-méthylpipéridin-4-yl)-4*H*-chromen-4-one (alvocidib) et des sels pharmaceutiquement acceptables correspondants.

**4.** Combinaison pour une utilisation selon la revendication 1, l'inhibiteur de CDK 4/6 étant le palbociclib.

**5.** Combinaison pour une utilisation selon la revendication 4, le palbociclib étant administré en un dosage quotidien choisi dans le groupe constitué par 75 mg/jour, 100 mg/jour et 125 mg/jour.

**6.** Combinaison pour une utilisation selon la revendication 1, l'inhibiteur de CDK 4/6 étant le ribociclib.

**7.** Combinaison pour une utilisation selon la revendication 6, le ribociclib étant administré en un dosage quotidien choisi dans le groupe constitué par 200 mg/jour, 400 mg/jour et 600 mg/jour.

**8.** Combinaison pour une utilisation selon la revendication 1, l'inhibiteur de CDK 4/6 étant l'abémaciclib.

**9.** Combinaison pour une utilisation selon la revendication 8, l'abémaciclib étant administré en un dosage quotidien choisi dans le groupe constitué par 200 mg/jour, 300 mg/jour et 400 mg/jour.

**10.** Combinaison pour une utilisation selon la revendication 1, le *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide ou un sel pharmaceutiquement acceptable correspondant et l'inhibiteur de CDK 4/6 ou un sel pharmaceutiquement acceptable correspondant étant administrés éventuellement en tant que formulations séparées, ou en tant qu'une formulation unique, ou étant administrés séquentiellement, ou étant administrés simultanément.

**11.** Combinaison pour une utilisation selon la revendication 1, le *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide étant la forme de base libre de *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide.

**12.** Combinaison pour une utilisation selon la revendication 11, le *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide ou un sel pharmaceutiquement acceptable correspondant étant administré en tant que dosage unique ou en plusieurs dosages.

**13.** Combinaison pour une utilisation selon la revendication 1, le sel pharmaceutiquement acceptable de *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide étant une forme de sel de chlorhydrate de *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide.

**14.** Formulation pharmaceutique comprenant du *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide ou un sel pharmaceutiquement acceptable correspondant et un inhibiteur de CDK 4/6 ou un sel pharmaceutiquement acceptable correspondant.

**15.** Formulation pharmaceutique selon la revendication 14, comprenant une forme de base libre de *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide.

**16.** Formulation pharmaceutique selon la revendication 14, le sel pharmaceutiquement acceptable de *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide étant une forme de sel de chlorhydrate de *N*-(2-((6-(3-(2,6-dichloro-3,5-diméthoxyphényl)-1-méthyluréido)pyrimidin-4-yl)amino)-5-(4-éthylpipérazin-1-yl)phényl)acrylamide.

FIG. 1A

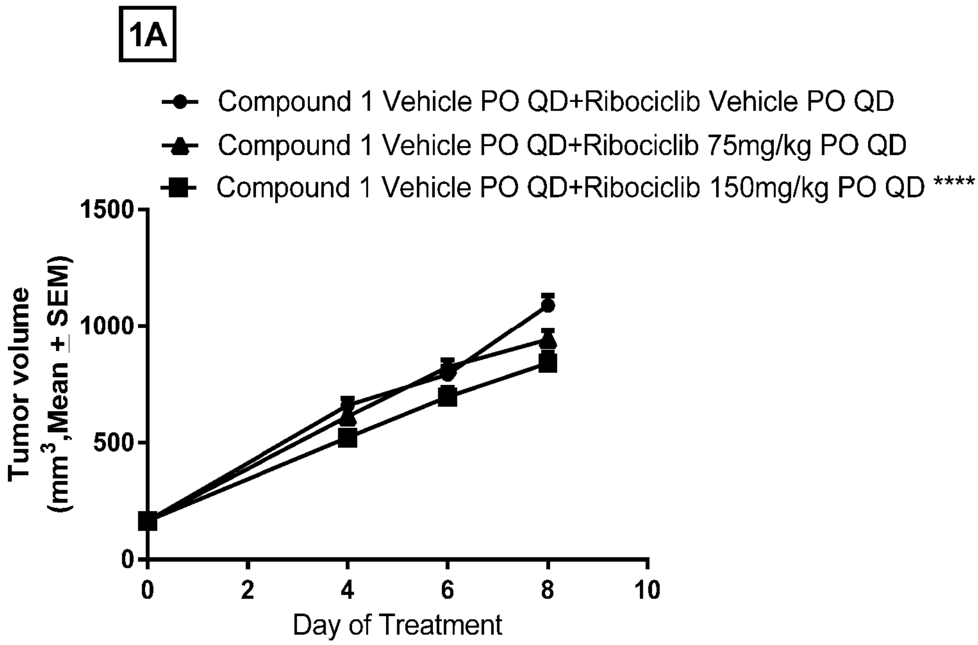
1A
Compound 1 Vehicle PO QD+Ribociclib Vehicle PO QD
Compound 1 Vehicle PO QD+Ribociclib 75mg/kg PO QD
Compound 1 Vehicle PO QD+Ribociclib 150mg/kg PO QD ****
Tumor volume
(mm3,Mean + SEM)
1500
1000
500
0
0
2
4
6
8
10
Day of Treatment

FIG. 1B

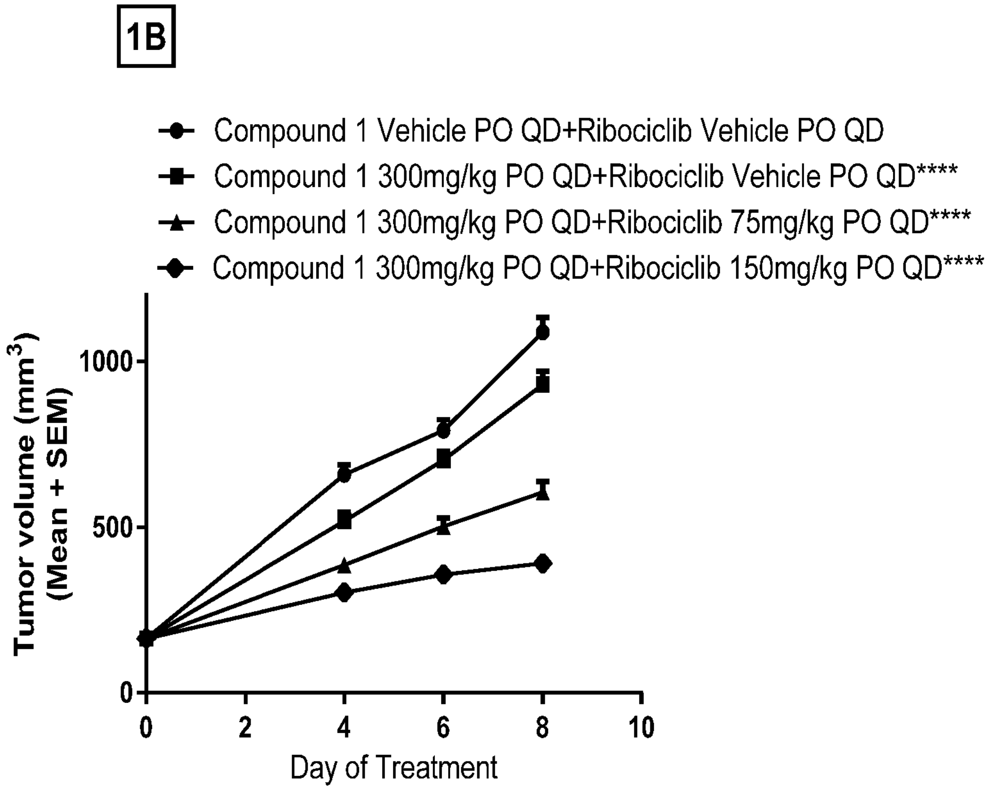
1B
Compound 1 Vehicle PO QD+Ribociclib Vehicle PO QD
Compound 1 300mg/kg PO QD+Ribociclib Vehicle PO QD****
Compound 1 300mg/kg PO QD+Ribociclib 75mg/kg PO QD****
Compound 1 300mg/kg PO QD+Ribociclib 150mg/kg PO QD****
Tumor volume (mm3)
(Mean + SEM)
1000
500
0
0
2
4
6
8
10
Day of Treatment

FIG. 1C

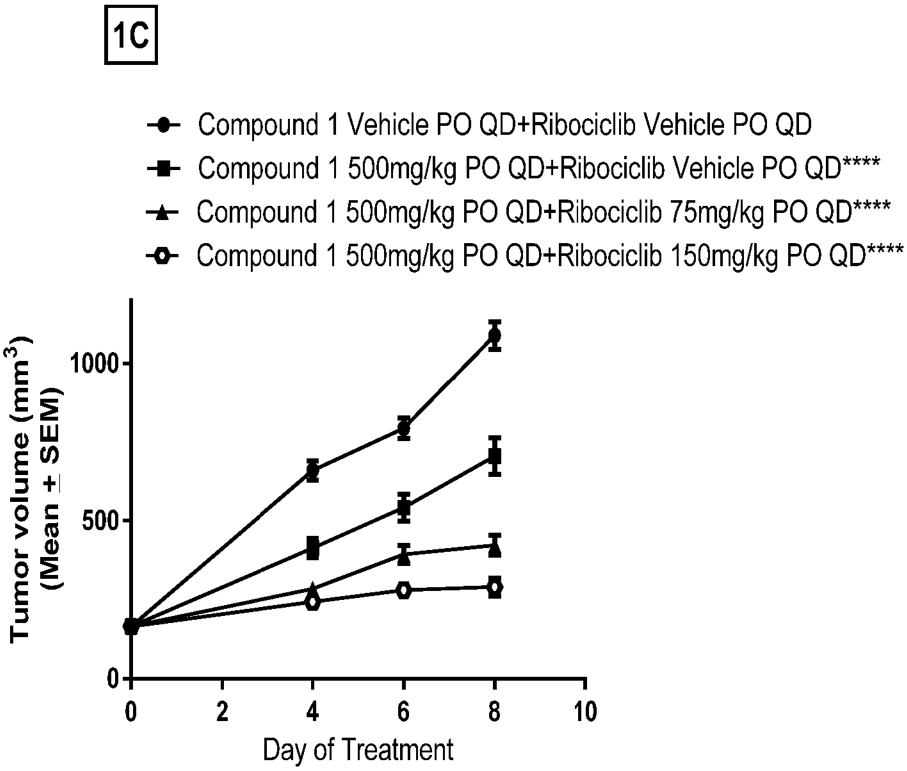
1C
Compound 1 Vehicle PO QD+Ribociclib Vehicle PO QD
Compound 1 500mg/kg PO QD+Ribociclib Vehicle PO QD****
Compound 1 500mg/kg PO QD+Ribociclib 75mg/kg PO QD****
Compound 1 500mg/kg PO QD+Ribociclib 150mg/kg PO QD****
Tumor volume (mm3)
(Mean ± SEM)
1000
500
0
0
2
4
6
8
10
Day of Treatment

FIG. 2A

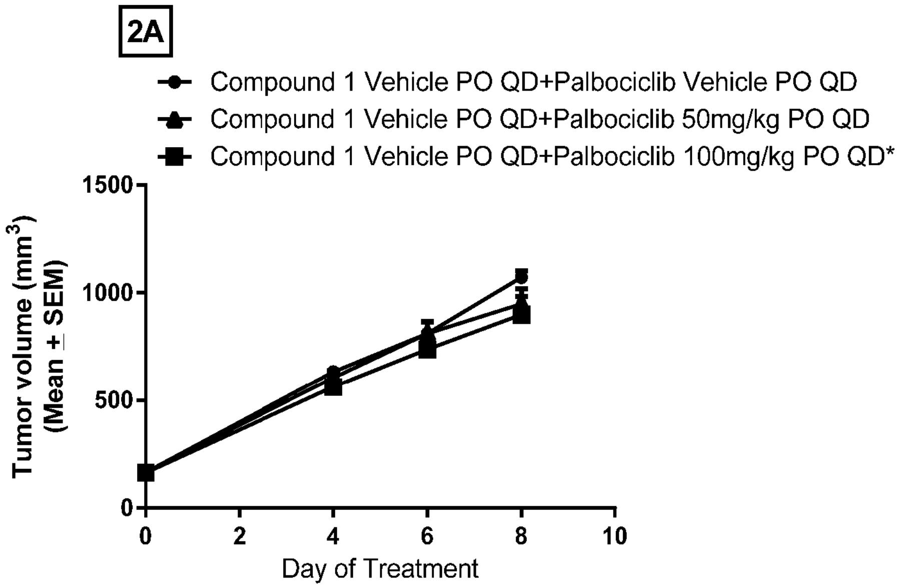
2A
Compound 1 Vehicle PO QD+Palbociclib Vehicle PO QD
Compound 1 Vehicle PO QD+Palbociclib 50mg/kg PO QD
Compound 1 Vehicle PO QD+Palbociclib 100mg/kg PO QD*
Tumor volume ($mm^3$) (Mean ± SEM)
1500
1000
500
0
0
2
4
6
8
10
Day of Treatment

FIG. 2B

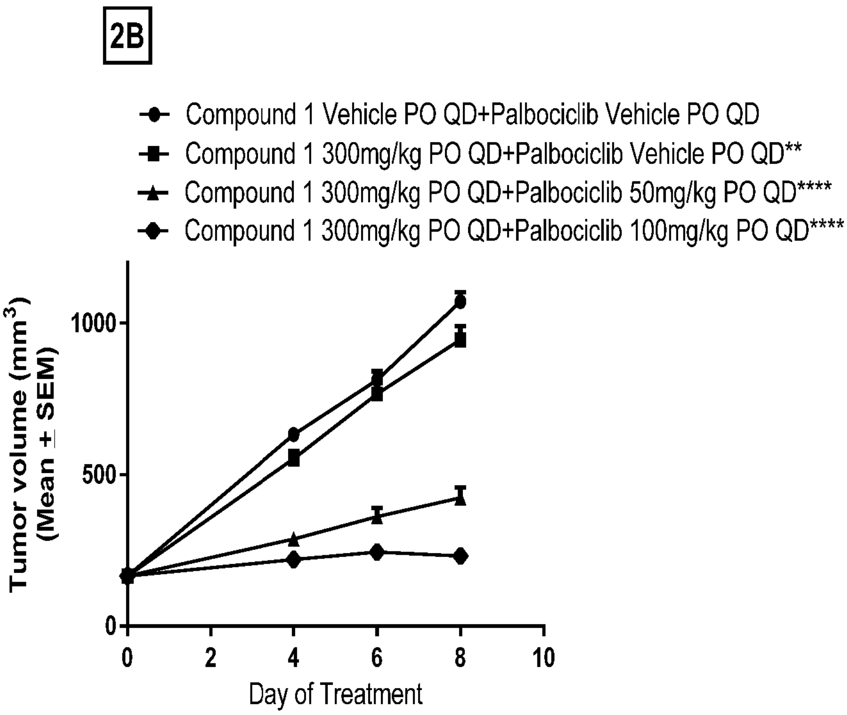
2B
Compound 1 Vehicle PO QD+Palbociclib Vehicle PO QD
Compound 1 300mg/kg PO QD+Palbociclib Vehicle PO QD**
Compound 1 300mg/kg PO QD+Palbociclib 50mg/kg PO QD****
Compound 1 300mg/kg PO QD+Palbociclib 100mg/kg PO QD****
Tumor volume ($mm^3$) (Mean ± SEM)
1000
500
0
0
2
4
6
8
10
Day of Treatment

FIG. 2C

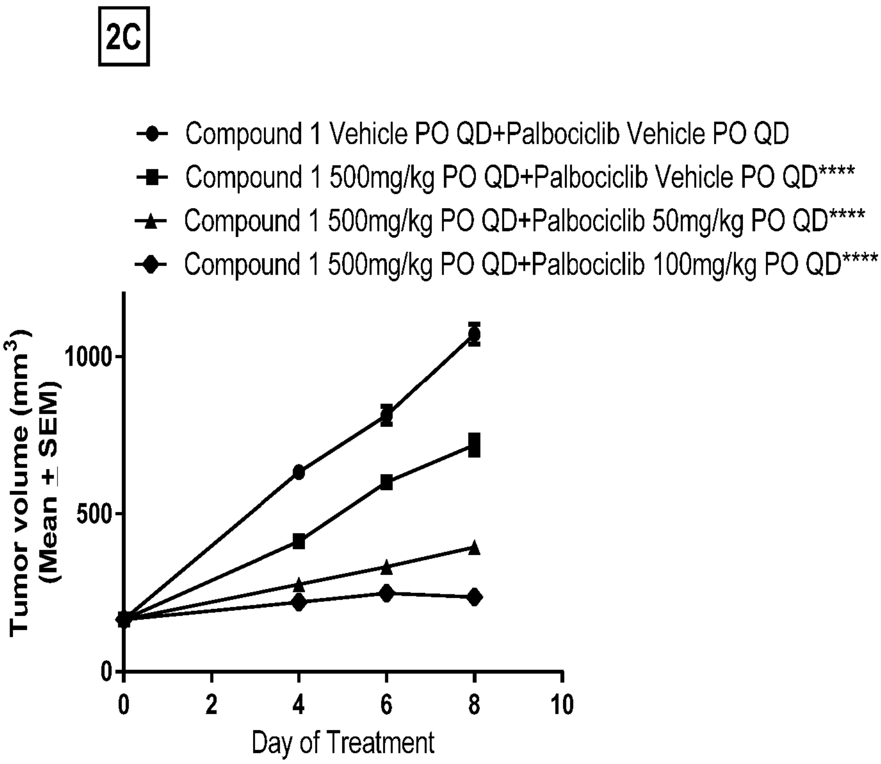
2C
Compound 1 Vehicle PO QD+Palbociclib Vehicle PO QD
Compound 1 500mg/kg PO QD+Palbociclib Vehicle PO QD****
Compound 1 500mg/kg PO QD+Palbociclib 50mg/kg PO QD****
Compound 1 500mg/kg PO QD+Palbociclib 100mg/kg PO QD****
Tumor volume (mm³)
(Mean ± SEM)
1000
500
0
0
2
4
6
8
10
Day of Treatment

FIG. 3A

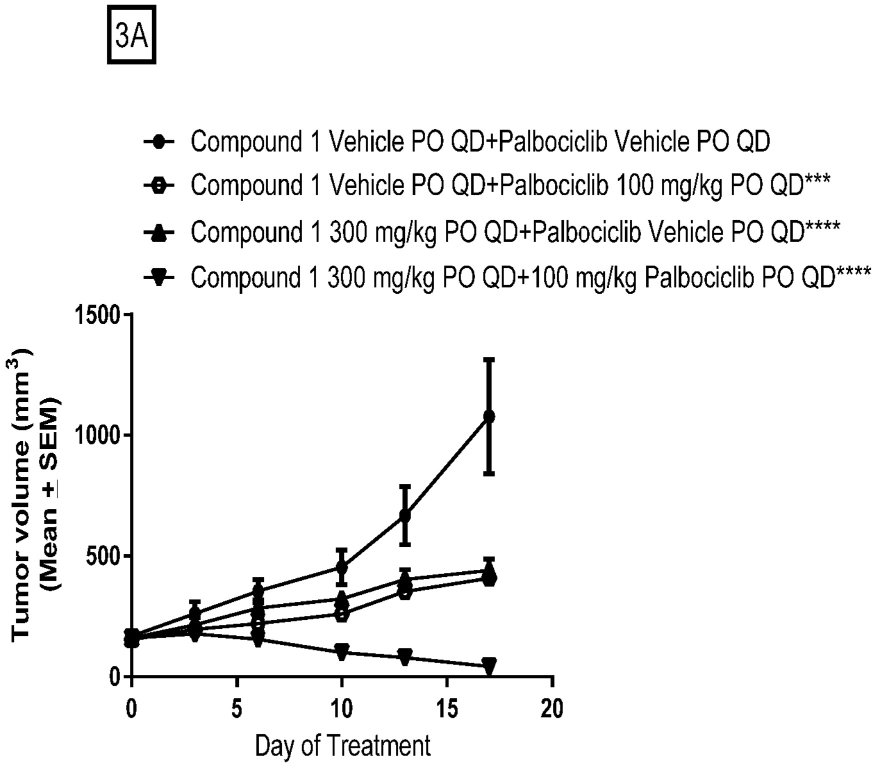
3A
Compound 1 Vehicle PO QD+Palbociclib Vehicle PO QD
Compound 1 Vehicle PO QD+Palbociclib 100 mg/kg PO QD***
Compound 1 300 mg/kg PO QD+Palbociclib Vehicle PO QD****
Compound 1 300 mg/kg PO QD+100 mg/kg Palbociclib PO QD****
Tumor volume (mm3) (Mean ± SEM)
1500
1000
500
0
0
5
10
15
20
Day of Treatment

FIG. 3B

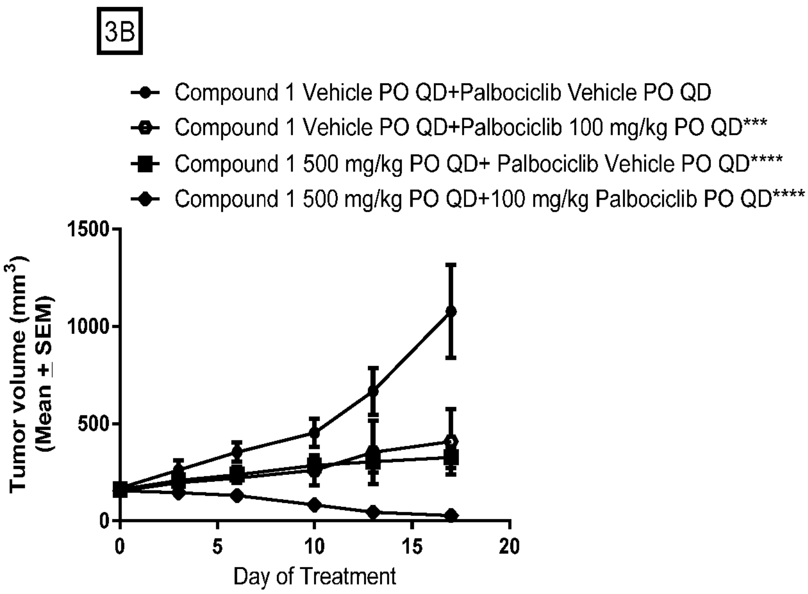
3B
Compound 1 Vehicle PO QD+Palbociclib Vehicle PO QD
Compound 1 Vehicle PO QD+Palbociclib 100 mg/kg PO QD***
Compound 1 500 mg/kg PO QD+ Palbociclib Vehicle PO QD****
Compound 1 500 mg/kg PO QD+100 mg/kg Palbociclib PO QD****
Tumor volume (mm3) (Mean ± SEM)
1500
1000
500
0
0
5
10
15
20
Day of Treatment

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015057938 A
- US 6936612 B
- US 7208489 B
- US 7456168 B
- US 20120115878 A
- WO 2007140222 A
- WO 2012061156 A
- WO 2011130232 A
- WO 2011101417 A
- WO 2016110224 A
- US 20100160340
- WO 2016025650 A
- US 20160220569 A
- WO 2014144326 A
- WO 2014144847 A
- WO 2016040848 A
- WO 2005012256 A
- WO 2006077424 A
- WO 2006077426 A
- WO 2008001101 A
- WO 2006077425 A
- WO 2006077428 A
- WO 2008007113 A
- WO 2008007122 A
- WO 2008009954 A
- US 2014163052
- WO 2012129344 A
- US 2011189175
- US 2011189175 A
- WO 2000044362 A
- WO 2001041747 A
- WO 2001053293 A
- WO 2001053294 A
- WO 2002022133 A
- WO 2007010946 A


### Non-patent literature cited in the description


- **LLOVET JM et al.** Advances in targeted therapies for hepatocellular carcinoma in the genomic era. *Nat. Rev. Clin Oncology,* 2015, vol. 12, 408-424
- **XIE MH et al.** FGF-19, a novel fibroblast growth factor with unique specificity for FGFR4. *Cytokine,* October 1999, vol. 11 (10), 729-35
- **SAWEY et al.** Identification of a therapeutic strategy targeting amplified FGF19 in liver cancer by Oncogenomic screening. *Cancer Cell,* 08 March 2011, vol. 19 (3), 347-58
- **RIVADENEIRA et al.** Proliferative Suppression by CDK4/6 Inhibition: Complex Function of the Retinoblastoma Pathway in Liver Tissue and Hepatoma Cells. *Gastroenterology,* 2010, vol. 138, 1920-1930
- **O'LEARY et al.** Treating Cancer with Selective CDK 4/6 Inhibitors. *Nat. Rev.,* 31 March 2016
- **J. SORRENTINO ; J. BISI ; P. ROBERTS ; J. STRUM.** G1T38, A Novel, Oral, Potent and Selective CDK 4/6 Inhibitor for the Treatment of RB Competent Tumors. *Abstract #2824 of the 2016 AACR Annual Meeting*
- Preclinical development of G1T38: A novel, potent and selective inhibitor of cyclin dependent kinases 4/6 for use as an oral antineoplastic in patients with CDK4/6 sensitive tumors. **BISI et al.** Oncotarget. Advance Publications, 15 March 2017
- **BISI et al.** Preclinical Characterization of G1T28: A Novel CDK4/6 Inhibitor for Reduction of Chemotherapy-induced Myelosuppression. *Mol. Cancer Ther.,* May 2016, vol. 15 (5), 783-93
- **STAHL et al.** Handbook of Pharmaceutical Salts: Properties, Selection, and Use. Wiley-VCH, 2002
- **BERGE et al.** *J. Pharm. Sci.,* 1977, vol. 66, 1
- Remington's Pharmaceutical Sciences. Mack Publishing Co,